(19) Europäisches Patentamt
European Patent Office
Office européen des brevets

(11) Publication number: **0 042 731**
**B1**

# (12) EUROPEAN PATENT SPECIFICATION

(45) Date of publication of patent specification: **05.12.84**

(21) Application number: **81302745.5**

(22) Date of filing: **18.06.81**

(51) Int. Cl.$^3$: **C 07 D 333/34,**
C 07 D 409/12,
C 07 D 417/12,
C 07 D 413/12, C 07 F 7/08,
A 01 N 43/00

## (54) Larvicides, insecticides, acaricides and miticides, processes for preparing them and compositions containing them.

(30) Priority: **21.06.80 GB 8020412**

(43) Date of publication of application:
**30.12.81 Bulletin 81/52**

(45) Publication of the grant of the patent:
**05.12.84 Bulletin 84/49**

(84) Designated Contracting States:
**BE CH DE FR GB IT LI LU NL SE**

(56) References cited:
**GB-A-1 459 571**
**GB-A-1 516 024**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT LI LU NL SE**

(72) Inventor: **Banks, Bernard Joseph**
**18 Victoria Avenue**
**St. Peters Broadstairs Kent (GB)**
Inventor: **Leeming, Michael Raymond Graves**
**2 Leycroft Close**
**Canterbury Kent (GB)**
Inventor: **Penrose, Alexander Ballingall**
**"Omega", Lower Street**
**Tilmanstone, Nr. Deal, Kent (GB)**
Inventor: **Walshe, Nigel Derek Arthur**
**157 Park Road**
**Sittingbourne Kent (GB)**

(74) Representative: **Wood, David John et al**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 042 731 B1

## Description

The invention relates to a series of thiophenesulphonamides with insecticidal activity, particularly against larval stages, and insect growth regulant activity. The compounds are particularly useful as blowfly larvicides. Some of the compounds have acaricidal activity and miticidal activity.

Eggs are laid by adult female insects on animal skins, and the larvae produced tend to burrow into the skins of the afflicted animals and thereby spoil the state of the skins, with the consequence, for example, that cattle hides and sheep skins and fleece intended for the manufacture of leather, sheepskin and woollen goods, respectively, are reduced in quality. Furthermore, the state of health and quality of the flesh of afflicted animals may be detrimentally affected. Certain insect larvae, for example, the larvae of blowflies which tend to live in sheep skin are capable of bringing premature death to the animal if present in sufficient abundance.

This invention provides compounds of the formula:

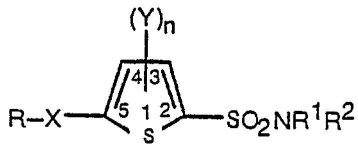

— (I)

and their alkali metal or non-toxic acid addition salts;
wherein:

(a) $R_1$ is hydrogen or lower alkyl;

and $R^2$ is lower alkyl, lower alkoxy, 2,2,2-trifluoroethyl, hydroxy-lower alkyl, 2-methoxyethoxymethyl, lower alkoxycarbonyl-lower alkyl, lower alkanoyloxy-lower alkyl, aryl-lower alkyl, aryloxy-lower alkyl, thiocyanatoacetyl, lower cycloalkyl-lower alkyl, lower cycloalkyl, lower cycloalkenyl, lower alkenyl, allenyl, lower alkynyl, $C_2$—$C_{16}$ alkanoyl, halo-lower alkanoyl, aryl-lower alkanoyl, N,N-di(lower alkyl)sulfamoyl, 1,2,3,4-tetrahydronaphthyl, arylthio, aryl or aroyl;

or $R^1$ and $R^2$ together with the nitrogen atom to which they are attached form a saturated 5- or 6-membered heterocyclic group optionally containing a further heteroatom selected from O, S and N, said N atom bearing a hydrogen atom or a lower alkyl or aryl group, said heterocyclic group optionally being substituted on a carbon atom by 1 or 2 lower alkyl groups;

(b) X is O, S, SO or $SO_2$;

(c) Y is halogen, lower alkyl, tri(lower alkyl)silyl, lower alkoxy, or a group of the formula $R^3S$— or $R^3SO_2$— wherein $R^3$ is lower alkyl or aryl; n is 0 or 1; *and*

(d) R is a lower alkyl, lower alkenyl, lower cycloalkyl, allenyl, lower alkoxy-lower alkyl, aryl-lower alkyl, aryl, heteroaromatic or heteroaromatic-phenyl group, with the proviso that when X is $SO_2$, R can also be —$NR^1R^2$ wherein $R^1$ and $R^2$ are as defined above.

The term "lower" as applied to an alkyl, alkoxy, alkanoyl, alkenyl or alkylnyl group means that said group has up to 6, preferably up to 4, carbon atoms in a straight, or where appropriate a branched, chain. Lower as applied to cycloalkyl means that said cycloalkyl group has from 3 to 7 carbon atoms. Lower cycloalkenyl groups have from 4 to 7 carbon atoms.

"Halogen" means fluorine, chlorine, bromine or iodine.

"Aryl" as used in this specification (including as a part of "aroyl") means unsubstituted phenyl, unsubstituted naphthyl, or substituted phenyl. "Substituted phenyl"is preferably either (a) phenyl substituted by 1 or 2 substituents selected from lower alkyl, lower alkoxy, hydroxy, halogen, trifluoromethyl, cyano, amino, di(lower alkyl)amino, nitro, lower alkylthio, lower alkylsulphinyl, lower alkylsulphonyl, thiocarbamoyl, isothiocyanato, methylenedioxy, sulphamoyl, N,N-di(lower alkyl)sulphamoyl, and N-(lower alkyl)carbamoyloxy (b) pentafluorophenyl (c) biphenylyl or (d) phenyl monosubstituted by phenoxy, cyanophenoxy, halophenoxy, nitrophenoxy, dinitrophenoxy, pentafluorophenoxy or halophenylthio.

The preferred heteroaromatic groups are thienyl, pyridyl, imidazolyl, benzthiazolyl, benzoxazolyl, pyrimidinyl, quinolyl, thiazolyl or pyridazinyl, optionally containing one or two substituents selected from lower alkyl, lower alkoxy and halogen.

When $R^1$ and $R^2$ together with the nitrogen atom to which they are attached form a heterocyclic group as defined above, this group is preferably a 1-pyrrolidinyl, piperidino, morpholino or piperazino group, said morpholino group optionally being substituted by one or two methyl groups, and said piperazino group optionally being substituted in the 4-position by a phenyl group which may itself be substituted by 1 or 2 halogen or $CF_3$ groups.

The non-toxic acid addition salts of succifiently basic compounds of the invention include for example the hydrochloride, hydrobromide, acetate, fumarate, lactate, tartrate and citrate salts. Preferred alkali metal salts are the sodium and potassium salts.

In the preferred compounds:

(a) $R^1$ is hydrogen or $C_1$—$C_4$ alkyl;

and $R^2$ is $C_1$—$C_4$ alkyl, methoxy, 2,2,2-trifluoroethyl, hydroxymethyl, 2-methoxyethoxymethyl, $C_1$—$C_4$ alloxycarbonylmethyl, $C_2$—$C_5$ alkanoyloxymethyl, benzyl; benzyl mono- or di-substituted on the phenyl ring portion by $C_1$—$C_4$ alkyl or halogen or mono-substituted by methylenedioxy; pentafluorophenylmethyl, phenethyl, phenoxy-($C_1$—$C_4$ alkyl), thiocyanatoacetyl, cyclopropylmethyl, $C_3C_6$ cycloalkyl, cyclohexenyl, allyl, allenyl, propargyl, $C_1$—$C_{16}$ alkanoyl, haloacetyl, phenylacetyl, N,N-dimethylsulphamoyl, 1,2,3,4-tetrahydronaphth-1-yl, phenylthio, benzoyl optionally mono- or di-substituted by $C_1$—$C_4$ alkyl, $C_1$—$C_4$ alkoxy or halogen; or phenyl;

or $R^1$ and $R^2$ taken together with the nitrogen atom to which they are attached represent a 1-pyrrolidinyl, piperidino, morpholino or piperazino group, said morpholino group optionally being substituted by 1 or 2 methyl groups, and said piperazino group optionally being substituted in the 4-position by a phenyl group which may itself be substituted by 1 or 2 halogen or $CF_3$ groups;

(b) X is O, S, SO or $SO_2$;

(c) Y is halogen, $C_1$—$C_4$ alkyl, trimethylsilyl, $C_1$—$C_4$ alkoxy or a group of the formula $R^3S$— where $R^3$ is $C_1$—$C_4$ alkyl or halophenyl; n is 0 or 1; and

(d) R is $C_1$—$C_4$ alkyl, allyl, $C_3$—$C_6$ cycloalkyl, allenyl, methoxymethyl, phenethyl, naphthyl, biphenylyl, thienyl, pyridyl, halopyridyl, dihaloimidazolyl, quinolyl, benzoxazolyl, benzthiazolyl, methoxypyridazinyl; phenyl; phenyl mono- or di-substituted by $C_1$—$C_4$ alkyl, $C_1$—$C_4$ alkoxy, halogen, cyano, trifluoromethyl, hydroxy, sulphamoyl, N,N-dimethylsulfamoyl, amino, dimethylamino, nitro, methylthio, methylsulphinyl, methylsulphonyl, N-methylcarbamoyloxy, isothiocyanato, or thiocarbamoyl; or phenyl monosubstituted by phenoxy, pentafluorophenoxy, cyanophenoxy, nitrophenoxy, dinitrophenoxy, halophenoxy, halophenylthio or 4-methylthiazol-2-yl; with the proviso that when X is $SO_2$, R can also be —$NR^1R^2$ wherein $R^1$ is $C_1$—$C_4$ alkyl, and $R^2$ is $C_1$—$C_4$ alkyl, cyclopropyl or allyl, or $R^1$ and $R^2$ taken together with the nitrogen atom to which they are attached represent a morpholino group optionally substituted by 1 or 2 methyl groups.

Another preferred group of compounds of the formula (I) are those wherein:—

(a) $R^1$ is hydrogen or lower alkyl;

and $R^2$ is lower alkyl, lower alkoxycarbonyl-lower alkyl, lower alkanoyloxy-lower alkyl, aryl-lower alkyl, lower cycloalkyl-lower alkyl, lower cycloalkyl, lower cycloalkenyl, lower alkenyl, allenyl, lower alkynyl, lower alkanoyl, halo-lower alkanoyl, aryl, aroyl, aryl-thio or lower alkoxy; or $R^1$ and $R^2$ together with the nitrogen atom to which they are attached form a saturated 5- or 6-membered heterocyclic group optionally containing a further heteroatom selected from O, S and N, said N atom bearing a hydrogen atom or a lower alkyl or aryl group, said heterocyclic group optionally being substituted on a carbon atom by 1 or 2 lower alkyl groups;

(b) X is O, S, SO or $SO_2$;

(c) Y is halogen, lower alkyl, lower alkoxy, tri(lower alkyl)silyl or a group of the formula $R^3S$— or $R^3SO_2$— wherein $R^3$ is lower alkyl or aryl; n is 0 or 1; *and*

(d) R is a lower alkyl, lower alkenyl, allenyl, lower alkoxy-lower alkyl, aryl-lower alkyl, aryl or heteroaromatic group, with the proviso that when X is $SO_2$ may also be —$NR^1R^2$ wherein $R^1$ and $R^2$ are as defined above;

"aryl" means unsubstituted phenyl, unsubstituted naphthyl, or phenyl substituted by 1 or 2 substituents selected from lower alkyl, lower alkoxy, hydroxy, halogen, trifluoromethyl, cyano, nitro, lower alkylthio, lower alkylsulphinyl, lower alkylsulphonyl, and N,N-di(lower alkyl)sulphamoyl; and "lower" is as previously defined.

The most preferred compounds have the formula:—

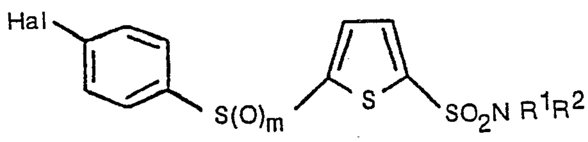

--- (IA)

wherein $R^1$ is hydrogen or $C_1$—$C_4$ alkyl, $R^2$ is $C_1$—$C_4$ alkyl, $C_3$—$C_6$ cycloalkyl, $C_2$—$C_4$ alkanoyl or aroyl (preferably benzoyl), "Hal" is Cl or Br, and m is 0 or 2;

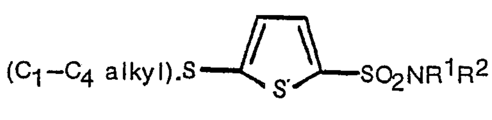

--- (IB)

wherein $R^1$ and $R^2$ are as defined for formula (IA) above.

The most preferred individual compounds are as follows:—

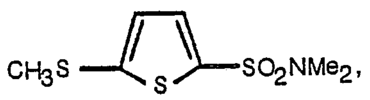

or 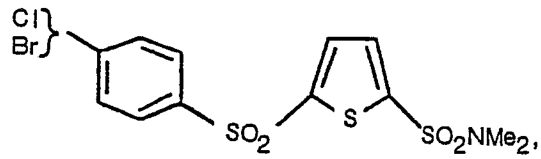

or 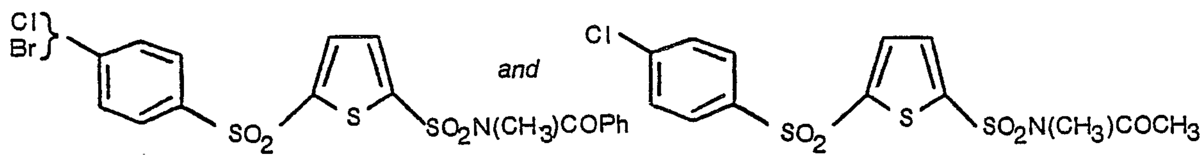

and

The compounds of the invention when used for treating ectoparasitic infections of animals such as sheep and cattle are suitably administered in the form of dusts or wettable powders, or as dips, "pour-on" formulations or sprays comprising an aqueous emulsion of an emulsifiable concentrate. The latter may be, for example, a 1—40% (g/100 ml) solution of the compound in a non-toxic organic solvent containing an emulsifying agent, and this may be diluted with water to give a concentration of the compound in the aqueous medium of from 0.01 to 0.5% w/v (g/100 ml), or approximately 100 to 5000 p.p.m. Suitable solvents include toluene, xylene and petroleum oil or an alkylated naphthalene.

The volatile solvents, e.g. toluene and xylene, evaporate after spraying to leave a deposit of the active ingredient. Suitable emulsifiers, which can be cationic, anionic or non-ionic as is well known to those skilled in the art, include ordinary soaps (anionic), lauryl pyridinium chloride (cationic) and polyoxyethylene lauryl ethers (non-ionic), the latter being, for example, a reaction product of ethylene oxide (10 moles) with dodecyl alcohol (1 mole). The made-up spray or dip may be an emulsion or suspension.

A dust may be made by mixing the appropriate amount of active ingredient with a diluent or carrier such as talc, clay, calcite, pyrophyllite, diatomaceous earth, walnut shell flour, silica gel, hydrated alumina or calcium silicate to afford a concentration of active ingredient of from about 0.25 to about 4% by weight. As an alternative method of preparation, the diluent or carrier is mixed with a solution of the active ingredient in a volatile organic solvent, e.g. benzene or acetone. The solvent is then removed by evaporation and the mixture ground.

Wettable powders may be made by adding suitable wetting agents and conditioning agents to the dusts. They may suitably contain from about 25% to about 75% w/v of active ingredient.

For the control of nuisance flies breeding in dung, the compounds may be incorporated into the animal feed.

Administration in the form of prolonged-release formulations is also possible.

The compounds may also be administered in combination with a conventional ectoparasiticidal agents such as organophosphates, carbamates, organochlorines, pyrethroids, formamidines, triazapentadienes, triazinethiones or thioureas.

In one test 2—4 day old females of the World Health Organisation standard fully susceptible strain of *Musca domestica* (common housefly) are anaesthetised with carbon dioxide and then each contacted on the dorsal surface of the thorax with one microgram of the test compound contained in 1 microlitre of solution, using e.g. acetone, methanol or methyl ethyl ketone as the solvent. The flies are then maintained for 24 hours in gauze-covered pots at 25°C and at about 50% relative humidity with a cotton wool pad moistened with sugar solution as a source of food placed on the gauze. Contacted with an equivalent volume of solvent only are the same number of anaesthetised control flies. At the end of the 24 hours period the mortality is noted and recorded as a percentage, after correction for any mortality amongst the controls. The result gives a measure of the effectiveness of the test compound applied topically.

In order to test the effectiveness of the compounds when used as bait for adult blow-flies, two groups of adult female *Lucilia sericata* or *cuprina* are maintained for 24 hours in pots each containing sugar and a pad of wet cotton wool as a free water supply, one of the sugar supplies being impregnated with test compound to the extent of 100 p.p.m. The mortality is noted as in the previous test and recorded as a corrected percentage.

Larvicidal properties are investigated by maintaining test and control groups of *Lucilia sericata* or *cuprina* (blowfly) larvae in separate test tubes, each containing filter paper partially soaked in calf serum serving as food and plugged with cotton wool. The filter paper in the test tube containing the test larvae is additionally impregnated with the compound under investigation to the extent of a 100 mg/$m^2$ deposition. Both test tubes are stored with the top part only in a strong light so as to induce the larvae to stay in the lower part of the tubes in contact with the filter paper through exploitation of their aversion to light. As in previous tests, mortality is noted and recorded as a corrected percentage. The finding of substantial numbers of test larvae on the illuminated plug suggests that the test compound has marked repellent properties.

In addition to percentage effectiveness figures, $LD_{90}$ results can be obtained from dose response measurements using any of the afore-described tests.

The compounds of the formula (I) in which X is S, except those in which R is an aryl, heteroaryl, or heteroaryl-phenyl group, have acaricidal and miticidal properties.

To assess acaricidal activity, in one test, five freshly collected, fully engorged *Boophilus microplus* adult female ticks are used for each acaricidal compound. Using a micro-pipette 10 microlitres of a solution containing 10 micro-grams of the acaricidal compound in ethanol or acetone, is applied to the dorsal surface of each of the ticks. The treated ticks are placed in weighed 1" × 2" glass vials, weighed and stored at 26°C and 80% + R.H. in plastic boxes for two weeks. The ticks are then removed from the vials and the vials weighed to give the weight of eggs laid by the ticks. Any reduction in the egg laying of the treated ticks is calculated as a percentage of the eggs laid by untreated control ticks.

The eggs are returned to the incubator for a further 3 weeks after which time the percentage of eggs hatching is estimated.

The percentage reduction in the anticipated reproduction of the ticks is calculated using the weight of eggs laid and the percentage of eggs hatching.

The test may be repeated using smaller amounts of the acaricidal compound.

In another test, using a pipette 0.5 ml of a solution containing 0.5 mg of the acaricidal compound in ethanol or acetone is spread evenly on to a Whatman No. 1 filter paper 8 cm × 6.25 cm (50 sq. c.m.) to give a dosage of 100 $mg/m^2$.

The treated paper is allowed to dry at room temperature, folded with the treated surface inside and the two short edges sealed with a crimping machine. The open ended envelope is placed in a 1 lb Kilner jar containing damp cotton wool in a plastic pot and stored in an incubator at 26°C for 24 hours. 20—50 *Boophilus microplus* larvae, which had hatched 8—14 days previously, are placed in the envelope using a small spatula.

The open end is then crimped to form a sealed packet. The treated paper containing the larvae is returned to the Kilner jar and kept for a further 48 hours in the incubator. 20—50 larvae are placed similarly in an untreated paper envelope to act as controls. At the end of the 48 hour test period the mortality is noted and recorded as a percentage after correction for any mortality among the untreated control ticks.

The test may be repeated using smaller amounts of the acaricidal compound.

In addition to percentage effectiveness figures, $LD_{90}$ results can be obtained from dose response measurements using any of the aforedescribed tests.

Activity against *Haemaphysalis longicornus nymphs* may be measured in a similar manner to the above larvae test.

In order to test the compounds for insect growth regulant activity, 24 hour old *Musca domestica* (housefly) larvae are added to a nutrient gel impregnated with the test compound at a dose concentration of 100 μg/ml. A typical nutrient gel consists of agar 10 gm., dessicated yeast 50 gm., skimmed milk powder 50 gm, and water 500 ml. Assessment of the activity is made by observing larval growth at 24 hours and 5 days and comparing state with that of the controls. Pupal state is observed and pupae counted on day 12. Enclosed flies are counted on day 20 and results are calculated against control figures. A compound giving 100% kill is further tested by dose response.

The test may also be carried out in a similar fashion using *Lucilia sericata* (sheep blowfly) larvae.

The compounds of the invention may be prepared by a number of routes, including the following:—

(1) The compounds of the formula (I) in which X is S may be prepared by the folowing reaction scheme:—

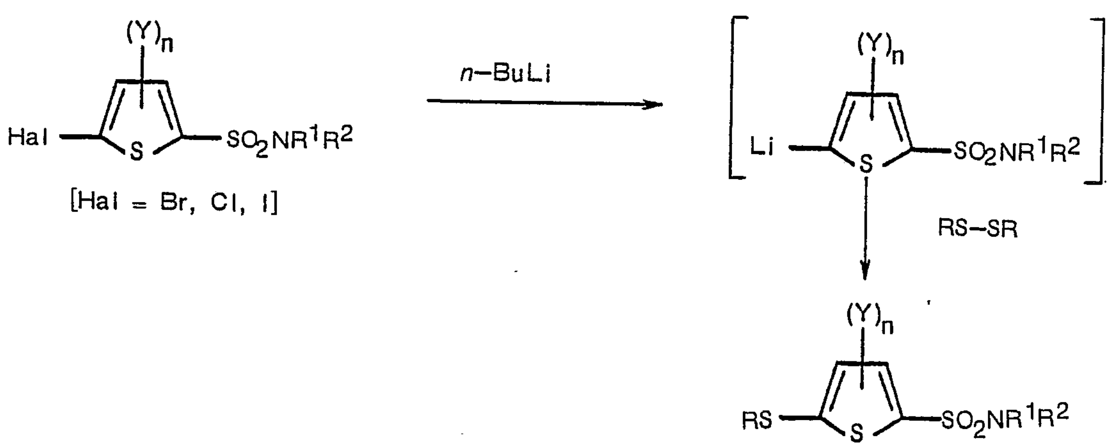


Preferably the bromo-derivative is used.

In a typical procedure the halo-thiophenesulphonamide in a suitable solvent, e.g. anhydrous ether,

at a low temperature (not more than —60°C) and under a dry nitrogen atmosphere, has added to it the *n*-butyllithium (or other alkyllithium) at such a rate that the internal temperature does not exceed —60°C. After stirring for a short period of time, the disulphide is added and the reaction mixture is then allowed to warm to room temperature. The resulting product may then be isolated and purified by conventional procedures.

(2) The compounds of the formula (I) in which X is S or O and R is an aryl or heteroaryl group may be prepared by the following reaction scheme:—

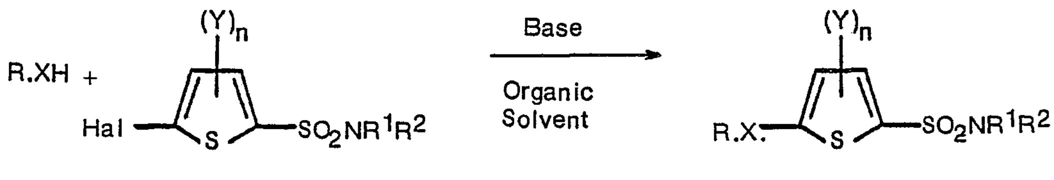


[Hal = Br, Cl, I]

The preferred base/solvent combination when X is S is dimethylformamide/NaOH/$H_2O$, and when X is O, hexamethylphosphoric triamide/anhydrous potassium carbonate. Typically the reactants are heated together in the base/solvent mixture at from 60—130°C under an atmosphere of nitrogen until the reaction is substantially complete, e.g. for from 5—25 hours. The product may then be isolated and purified by conventional procedures.

(3) The compounds of the formula (I) in which $R^2$ is other than an aryl group, and in which $R^1$ and $R^2$ do not form a ring, may be prepared as follows:—

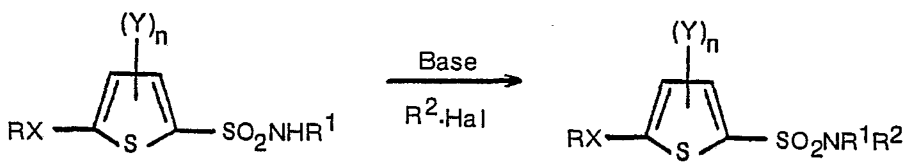


[Hal = Br, Cl, I]

Preferably "Hal" is Cl when $R^2$ is an alkanoyl, halo-alkanoyl or aroyl group; otherwise "Hal" is generally Br.

In a typical procedure a solution of the reactants in a suitable solvent, e.g. dimethylformamide, and in the presence of a base, e.g. sodium hydride, is stirred at room temperature (20°C) for a few hours, although in some cases heating may be necessary, e.g. to 70°C, to accelerate the reaction. The product may then be isolated and purified by conventional methods.

(4) The compounds of the formula (I) in which $R^1$ is a lower alkyl group may be prepared as follows:—

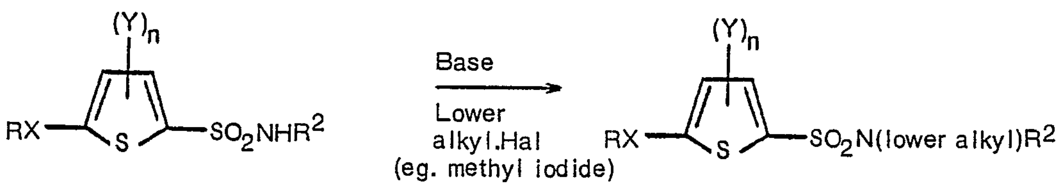


[Hal = Br, Cl, I]

The reaction may be carried out in a similar manner to (3) above.

Similarly compounds in which both $R^1$ and $R^2$ are lower alkyl groups may be prepared as follows:—

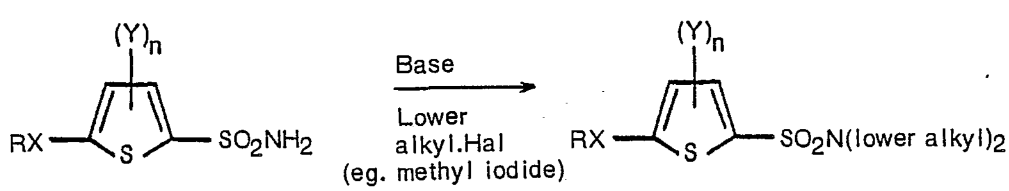

Generally a slight excess of the lower alkyl halide, i.e. more than 2 equivalents, is used. When X is $SO_2$ and R is $R^2NH$—, then trialkylation may occur to give

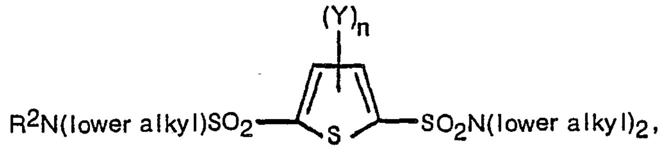

when an excess of alkylating agent is used.

(5) The compounds of the formula (I) in which X is S may be prepared as follows:

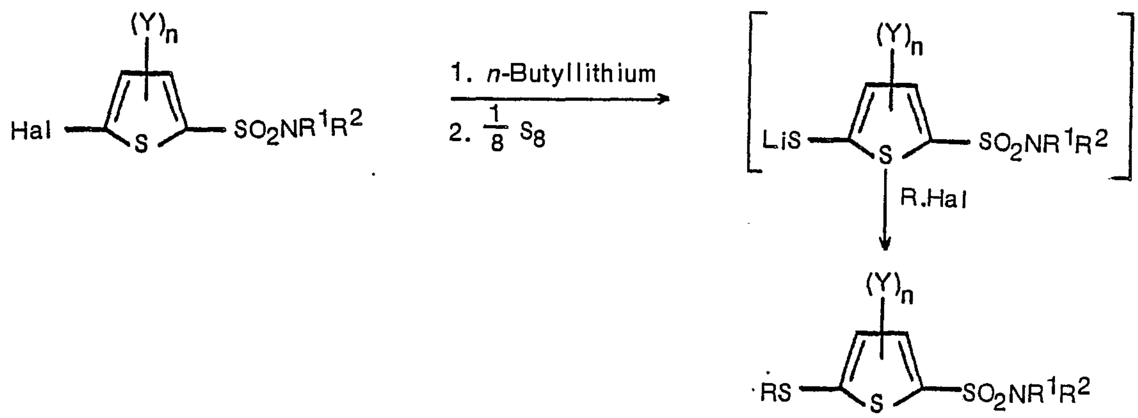


When R in "R.Hal" is an aryl group, generally a strongly electron withdrawing substituent (e.g. $NO_2$) must be present in the meta- or para-position to the halogen atom "Hal". Sometimes an electron-withdrawing substituent is similarly necessary when R is heteroaryl.

The reaction is typically carried out by reacting the halothiophenesulphonamide, which is preferably the bromothiophenesulphonamide, in a suitable solvent, e.g. anhydrous ether, with the n-butyllithium at a low temperature, generally below −60°C. Approximately one equivalent of sulphur is then added and the mixture stirred at this temperature for about an hour. After allowing the reaction mixture to warm to room temperature, the solvent is removed *in vacuo* and the resulting solid taken up in a suitable solvent, e.g. anhydrous dimethylformamide. The compound R.Hal is then added and the mixture stirred at room temperature for up to about 24 hours. The product may then be isolated and purified by conventional procedures.

(6) Compounds of the formula (I) in which X is SO can be prepared by the controlled oxidation of the corresponding compounds in which X is S. In a typical procedure, the appropriate compound in which X is S, in a suitable solvent, e.g. dichloromethane, is refluxed with about one equivalent of *m*-chloroperbenzoic acid for a few hours, e.g. for up to 5 hours. The product may then be isolated and purified by conventional procedures.

(7) Compounds of the formula (I) in which X is $SO_2$ can be prepared by the oxidation of the corresponding compounds in which X is S. This is typically carried out by oxidizing the starting material with an excess of hydrogen peroxide in glacial acetic acid under reflux for a few hours, e.g. for up to 5 hours. Again the product may then be isolated and purified by conventional procedures.

(8) Compounds of the formula (I) can also be prepared as follows:—

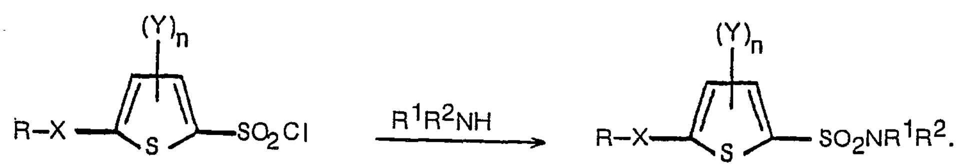


Generally the reaction is carried out at room temperature of lower in an inert organic solvent, e.g. dichloromethane. The product may be recovered by conventional procedures.

The starting materials may be prepared as follows:—

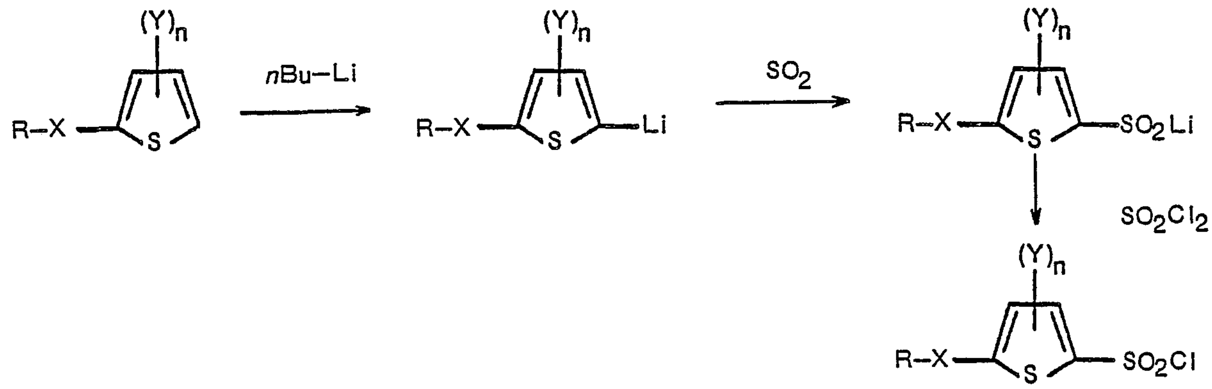


(9) The following route was also useful in a particular case:—

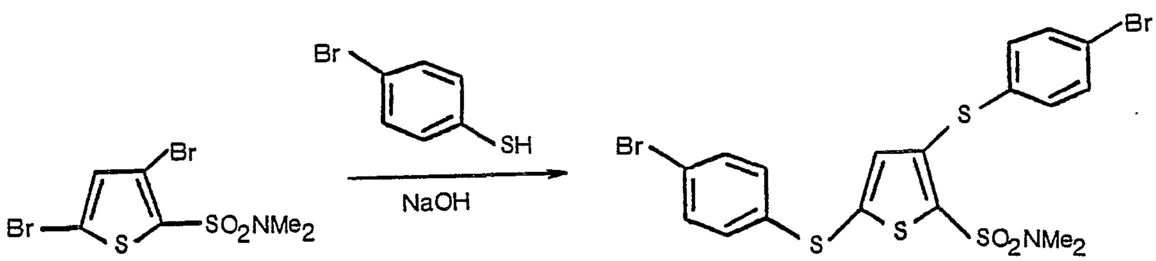


The details are given in Example 226.

(10) Certain compounds of the invention may be prepared from other compounds of the invention as follows:—

Compounds in which Y is $R^3S$—, lower alkyl, tri(lower alkyl)silyl or halogen may be prepared by the electrophilic substitution of the corresponding compound in which Y is H, e.g. using trimethylsilylchloride,

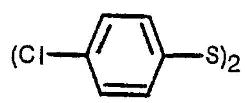 , $(CH_3S)_2$, $CH_3I$, $Br_2$ or

$Br(CH_2)_2Br$. [the use of $Br(CH_2)_2Br$ resulted in bromination].

Compounds in which Y is alkoxyphenyl or aryloxyphenyl may be prepared by the reaction of the corresponding hydroxyphenyl compound with the appropriate alkyl or aryl halide in the presence of a base, e.g. $K_2CO_3$. The aryl halide preferably has a strong electron-withdrawing substituent, e.g. nitro, in the *m*- or *p*-position.

Compounds having a phenyl group substituted by N-(lower alkyl)carbamoyloxy may be prepared by the reaction of the corresponding hydroxy-substituted compound with a lower alkyl isocyanate.

Compounds having an arylthiophenyl or aryloxyphenyl substituent may be prepared by the reaction of the corresponding halophenyl compound with the appropriate arylthiol or arylol in the presence of a base, e.g. $K_2CO_3$.

Compounds having a phenyl group substituted by dimethylamino may be prepared by the reaction of the corresponding fluorophenyl derivative with dimethylacetamide.

Compounds having a thiocarbamoyl substituent may be prepared by the reaction of the corresponding cyano compound with hydrogen sulphide and ammonia.

Compounds in which R is 4-methylthiazol-2-ylphenyl may be prepared as follows:—

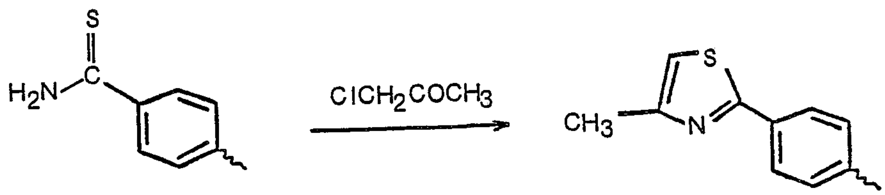


Compounds having an isothiocyanato substituent may be prepared from the corresponding amino-substituted compound by reaction with thiophosgene.

Compounds in which $R^2$ is $-COCH_2SCN$ may be prepared from the corresponding compound in which $R^2$ is $-COCH_2Cl$ or $-COCH_2Br$ by reaction with sodium or potassium thiocyanate.

Compounds in which $R^2$ is hydroxymethyl may be prepared from the corresponding compound in which $R^2$ is H by reaction with formalin.

All the above methods are conventional and are illustrated in the specific Examples hereinafter.

Salts of the compounds of the formula (I) can also be prepared by conventional procedures.

Many of the starting materials for the above procedures are known compounds (see e.g. British Patent Specifications Nos. 1459571, 1468111 and 1516024) or may be prepared by procedures analogous to those of the prior art.

The following Examples illustrate the invention. All temperatures are in °C and where quoted, n.m.r. values are reported on the $\delta$ scale relative to tetramethylsilane as internal standard:—

## Example 1

Preparation of 5-Methylthio-N,N-dimethyl-2-thiophenesulphonamide

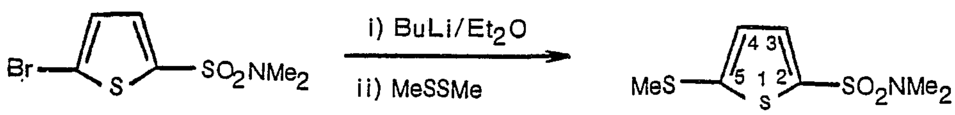


A stirred solution of 5-bromo-N,N-dimethyl-2-thiophenesulphonamide (42.2 g, 0.156 m) in anhydrous ether (1200 ml) was cooled to −70° under an atmosphere of dry nitrogen. *n*-Butyl lithium (110 ml, 1.6 m solution in hexane, 0.165 m) was added at such a rate that the internal temperature was maintained at below −60°. The resulting white suspension was stirred for 15 minutes then dimethyl disulphide (14.7 g, 14 ml, 0.156 m) was added. The cooling bath was removed and the reaction was allowed to warm to room temperature over a period of 2½ hours.

The reaction mixture was then poured into water (1000 ml) and the ethereal layer separated. This was then washed with water (500 ml, x3), soidum hydroxide solution (2N, 500 ml), water (500 ml), saturated sodium chloride solution (500 ml), and then dried ($MgSO_4$). The drying agent was removed by filtration and the filtrate evaporated *in vacuo* to give a pale brown oil.

This crude oil was purified by vacuum distillation at a pressure of 1 torr. The fraction having boiling range 180—186° was collected, yield of the title compound 29.6 g, 80%.

*Analysis %:—*

| | | | |
|---|---|---|---|
| Found: | C, 35.78; | H, 4.65; | N, 6.00 |
| Required: | C, 35.42; | H, 4.67; | N, 5.90 |

Examples 2—8

The following compounds were prepared by procedures similar to that of Example 1, starting from 5-bromo or 4-methyl-5-bromo-N,N-dimethyl-2-thiophenesulfonamide, the appropriate disulphide, and *n*-butyllithium:—

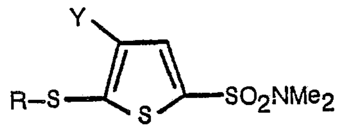

| Example No. | R | Y | M.P. (°C) | Analysis % or n.m.r. (Theoretical in brackets) C | H | N |
|---|---|---|---|---|---|---|
| 2 | Et | H | oil | 40.11<br>(38.22 | 4.99<br>5.21 | 5.96<br>5.97) |
| 3 | *i*-Pr | H | oil | 40.15<br>(40.72 | 5.55<br>5.70 | 5.51<br>5.28) |
| 4 | 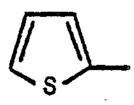 | H | oil | 39.13<br>(39.32 | 3.63<br>3.63 | 4.60<br>4.59) |
| 5 | Me | Me | B.P. t. 143–145° at 0.2 mmHg | 38.39<br>(38.22 | 5.20<br>5.21 | 5.17<br>5.57) |
| 6 | 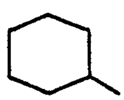 | H | 62–63° | 47.31<br>(47.18 | 6.24<br>6.27 | 4.59<br>4.59) |
| 7 | 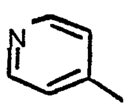 | H | 127–128° | 43.69<br>(43.98 | 4.02<br>4.03 | 9.12<br>9.33) |
| 8 | 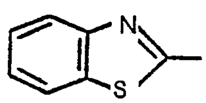 | H | 86–87° | 43.69<br>(43.79 | 3.48<br>3.39 | 7.86<br>7.86) |

Example 9

The following compound was prepared by a procedure similar to that of Example 1, starting from 5- bromo-N-(1,2,3,4-tetrahydronaphth-1-yl)-thiophene-2-sulphonamide, *p*-chlorophenyldisulphide, and *n*-butyllithium:—

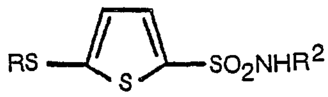

| Example No. | R | $R^2$ | M.P. (°C) | N.m.r. |
|---|---|---|---|---|
| 9 | 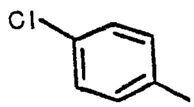 | 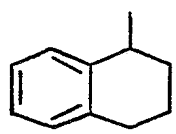 | gum | 7.6–6.8 (m,10N,Ar–H)<br>4.9–4.3 (m,2H,Ar–CH+NH)<br>2.9–2.5 (m,2H,Ar.$CH_2$),<br>2.0–1.5 (m,4H,$CH_2CH_2$) |

Example 10

Preparation of 5-Phenylthio-N,N-dimethyl-2-thiophenesulphonamide

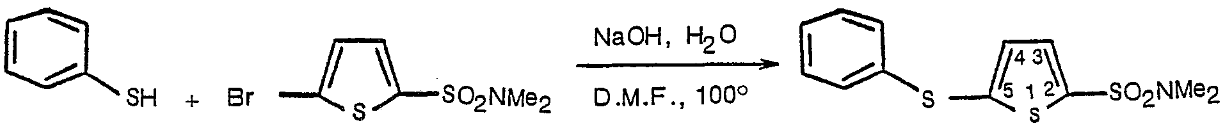


To a solution of benzenethiol (12.1 g, 0.11 m) in dimethylformamide (DMF) (180 ml) was added a solution of sodium hydroxide (4.4 g, 0.11 m) in water (20 ml). The mixture was stirred and 5-bromo-N,N-dimethyl-2-thiophenesulphonamide (27 g, 0.1 m) was added. The resulting solution was heated at 100° under an atmosphere of nitrogen for 6 hours. The reaction mixture was cooled and filtered. The filtrate was evaporated *in vacuo* and the residue partitioned between ether (500 ml) and hydrochloric acid (250 ml, 10%). The ether layer was separated, washed with sodium hydroxide solution (250 ml, 10%, ×2), water (250 ml), saturated sodium chloride solution (250 ml), and dried ($MgSO_4$). The drying agent was removed by filtration and the filtrate evaporated *in vacuo* to give an oil which solidified on standing.

The crude product was recrystallised from ethyl acetate/petroleum ether (b.p. 40°—60°) to give 5-phenylthio-N,N-dimethyl-2-thiophenesulphonamide as colourless crystals (24 g, 80%), m.p. 74—76°.

*Analysis %:—*

| | | | |
|---|---|---|---|
| Found: | C, 47.39 | H, 4.31; | N, 4.52. |
| Required: | C, 48.13; | H, 4.37; | N, 4.68. |

Examples 11—69

The following compounds were prepared by procedures similar to that of Example 10, starting from the appropriate aryl or heteroarylthiol and the appropriate 5-bromothiophene-2-sulphonamide.

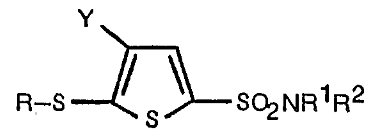

| Example No. | $R^1$ | $R^2$ | Y | R | M.P. (°C) | Analysis % or n.m.r. (Theoretical in brackets) C | H | N |
|---|---|---|---|---|---|---|---|---|
| 11 | Me | Me | H | 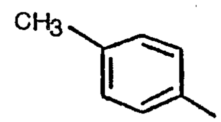 | 79–81° | 49.52 (49.81 | 4.89 4.82 | 4.65 4.47) |
| 12 | Me | Me | H | 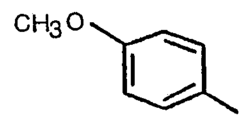 | 52–54° | 47.36 (47.39 | 4.62 4.59 | 4.45 4.25) |
| 13 | Me | Me | H | 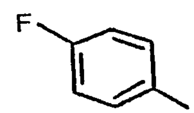 | 78–79° | 45.57 (45.40 | 3.72 3.81 | 4.49 4.41) |
| 14 | Me | Me | H | 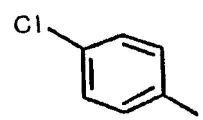 | 77–79° | 43.28 (43.17 | 3.64 3.62 | 4.18 4.19) |
| 15 | Me | Me | H | 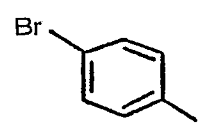 | 81° | 38.33 (38.09 | 3.18 3.20 | 3.81 3.70) |

| Example No. | $R^1$ | $R^2$ | Y | R | M.P. (°C) | Analysis % or n.m.r. (Theoretical in brackets) C | H | N |
|---|---|---|---|---|---|---|---|---|
| 16 | Me | Me | H | 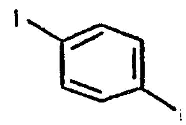 | 85–87° | 34.18 (33.88 | 2.77 2.84 | 3.36 3.29) |
| 17 | Me | Me | H | 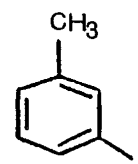 | 65° | 49.60 (49.81 | 4.79 4.82 | 4.59 4.47) |
| 18 | Me | Me | H | 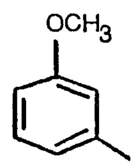 | 80–82° | 47.72 (47.39 | 4.58 4.59 | 4.38 4.25) |
| 19 | Me | Me | H | 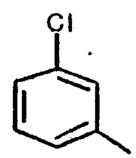 | ~ 70° | 43.02 (43.17 | 3.59 3.62 | 3.97 4.20) |
| 20 | Me | Me | H | 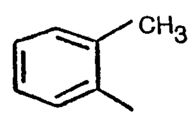 | 62–63° | 49.67 (49.84 | 4.75 4.79 | 4.25 4.42) |

| Example No. | R1 | R2 | Y | R | M.P. (°C) | Analysis % or n.m.r. (Theoretical in brackets) C | H | N |
|---|---|---|---|---|---|---|---|---|
| 21 | Me | Me | H | 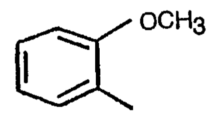 | 60–62° | 47.72 (47.39 | 4.58 4.59 | 4.38 4.25) |
| 22 | Me | Me | H | 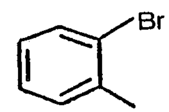 | 91–93° | 38.35 (38.09 | 3.13 3.17 | 3.79 3.70) |
| 23 | Me | Me | H | 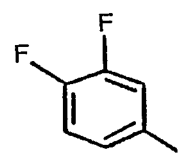 | oil | 2.75 (s, 6H) 6.9–7.5 (m,5H) | | |
| 24 | Me | Me | H | 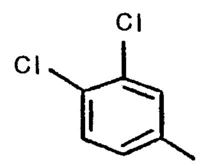 | 74–75° | 39.04 (39.13 | 2.98 3.01 | 3.42 3.80) |
| 25 | Me | Me | H | 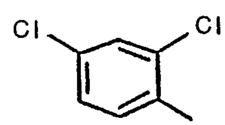 | oil | 38.81 (39.13 | 3.05 2.99 | 3.95 3.80) |

| Example No. | $R^1$ | $R^2$ | Y | R | M.P. (°C) | Analysis % or n.m.r. (Theoretical in brackets) C | H | N |
|---|---|---|---|---|---|---|---|---|
| 26 | Me | Me | H | 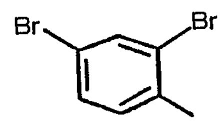 | 92–96° | 32.08 (31.52 | 2.30 2.42 | 3.02 3.06) |
| 27 | Me | Me | Br | 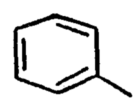 | 115–116° | 37.96 (38.09 | 3.41 3.19 | 3.64 3.70) |
| 28 | Me | Me | Br | 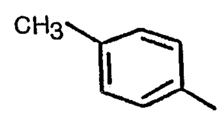 | 123–124° | 40.06 (39.80 | 3.56 3.60 | 3.74 3.57) |
| 29 | Me | Me | Br | 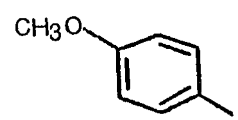 | 119–121° | 38.31 (38.24 | 3.51 3.45 | 3.34 3.43) |
| 30 | Me | Me | Br | 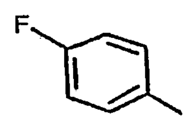 | 134–136° | 36.86 (36.36 | 2.98 2.79 | 3.58 3.53) |

| Example No. | $R^1$ | $R^2$ | Y | R | M.P. (°C) | Analysis % or n.m.r. (Theoretical in brackets) C | H | N |
|---|---|---|---|---|---|---|---|---|
| 31 | Me | Me | Br | 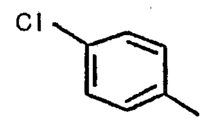 | 147–149° | 34.79 (34.91 | 2.68 2.68 | 3.83 3.39) |
| 32 | Me | Me | Br | 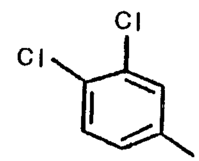 | 119–121° | 32.29 (32.22 | 2.59 2.25 | 3.24 3.13) |
| 33 | Me | Me | Me | 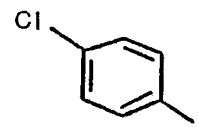 | 112.5–113.5° | 44.59 (44.88 | 3.99 4.06 | 4.00 4.03) |
| 34 | H | Me | H | 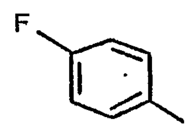 | 69–71° | 43.41 (43.55 | 3.18 3.32 | 4.85 4.62) |
| 35 | H | Me | H | 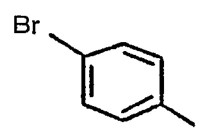 | 121° | 36.48 (36.26 | 2.86 2.76 | 4.15 3.84) |

| Example No. | $R^1$ | $R^2$ | Y | R | M.P. (°C) | Analysis % or n.m.r. (Theoretical in brackets) C | H | N |
|---|---|---|---|---|---|---|---|---|
| 36 | H | Et | H | 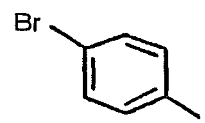 | 89° | 38.20 (38.09 | 3.04 3.20 | 3.56 3.70) |
| 37 | H | *i*-Pr | H | 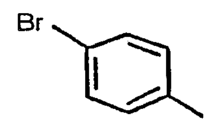 | ~ 90° | 39.43 (39.79 | 3.61 3.60 | 3.70 3.57) |
| 38 | Me | Et | H | 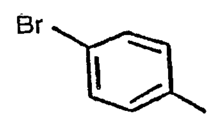 | 32–35° | 39.81 (39.79 | 3.33 3.60 | 3.90 3.57) |
| 39 | Me | *i*-Pr | H | 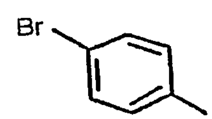 | 54–56° | 41.16 (41.38 | 3.91 3.97 | 3.37 3.45) |
| 40 | Me | *t*-Bu | H | 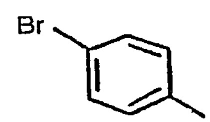 | 56–58° | 42.41 (42.85 | 4.10 4.32 | 3.31 3.33) |

| Example No. | $R^1$ | $R^2$ | Y | R | M.P. (°C) | Analysis % or n.m.r. (Theoretical in brackets) C | H | N |
|---|---|---|---|---|---|---|---|---|
| 41 | Me | allyl | H | 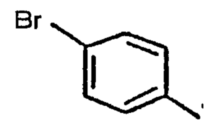 | 62–63° | 41.69 (41.58 | 3.39 3.49 | 3.33 3.46) |
| 42 | Me | cyclo-pentyl | H | 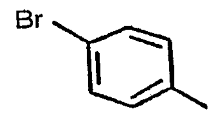 | 40–42° | 44.71 (44.44 | 4.60 4.20 | 3.29 3.24) |
| 43 | Me | cyclo-hexyl | H | 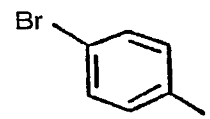 | 75–76° | 45.57 (45.73 | 4.47 4.52 | 3.05 3.14) |
| 44 | Me | phenyl | H | 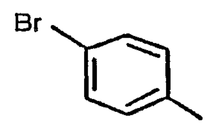 | 105° | 46.28 (46.36 | 3.15 3.20 | 3.15 3.18) |
| 45 | Me | benzyl | H | 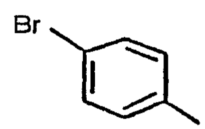 | 98.5° | 47.55 (47.57 | 3.57 3.55 | 2.68 3.08) |

| Example No. | $R^1$ | $R^2$ | Y | R | M.P. (°C) | Analysis % or n.m.r. (Theoretical in brackets) C | H | N |
|---|---|---|---|---|---|---|---|---|
| 46 | Et | Et | H | 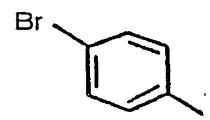 | 70° | 41.00 (41.38 | 3.83 3.97 | 3.28 3.45) |
| 47 | 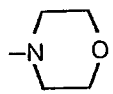 | | H | 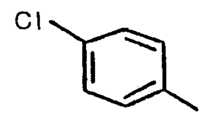 | 140–141° | 44.84 (44.80 | 3.76 3.82 | 3.79 3.71) |
| 48 | 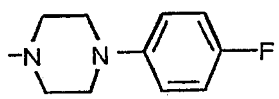 | | H | 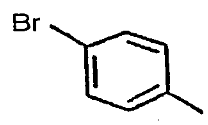 | 134° | 46.64 (46.78 | 3.48 3.53 | 5.59 5.46) |
| 49 | 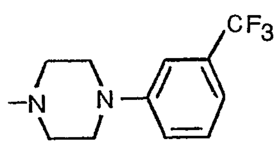 | | H | 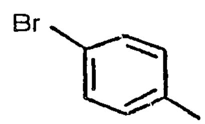 | 117° | 44.62 (44.76 | 3.26 3.22 | 5.00 4.97) |
| 50 | 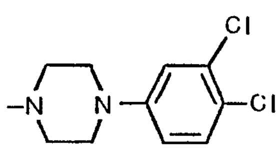 | | H | 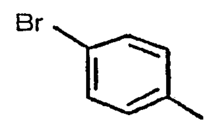 | 121–122° | 42.80 (42.56 | 3.03 3.04 | 5.34 4.96) |

| Example No. | $R^1$ | $R^2$ | Y | R | M.P. (°C) | Analysis % or n.m.r. (Theoretical in brackets) C | H | N |
|---|---|---|---|---|---|---|---|---|
| 51 | Me | Me | H | 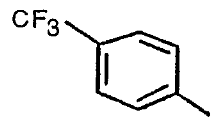 | 60–63° | 42.29 (42.49 | 3.29 3.29 | 3.65 3.81) |
| 52 | Me | Me | H | 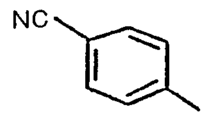 | 109–111° | 47.93 (48.12 | 3.68 3.73 | 8.79 8.64) |
| 53 | Me | Me | Br | 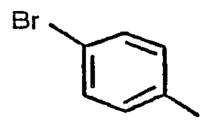 | 149–150° | 31.33 (31.52 | 2.29 2.42 | 3.10 3.06) |
| 54 | Me | Me | $CH_3$ | 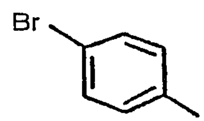 | 111–112° | 40.08 (39.79 | 3.53 3.60 | 3.54 3.57) |
| 55 | H | cyclo-propyl | H | 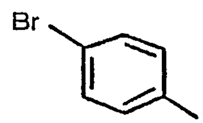 | 105–106° | 39.85 (40.00 | 3.02 3.10 | 3.80 3.59) |

| Example No. | $R^1$ | $R^2$ | Y | R | M.P. (°C) | Analysis % or n.m.r. (Theoretical in brackets) C | H | N |
|---|---|---|---|---|---|---|---|---|
| 56 | Me | Me | Br | 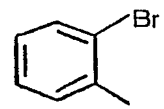 | 131° | 31.22 (31.52 | 2.29 2.42 | 3.07 3.06) |
| 57 | 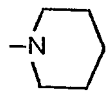 | | H | 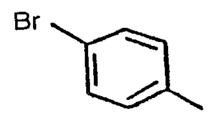 | 84° | 42.85 (43.06 | 3.69 3.85 | 3.40 3.35) |
| 58 | 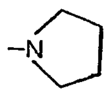 | | H | 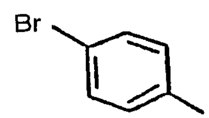 | 76° | 41.35 (41.58 | 3.38 3.49 | 3.64 3.46) |
| 59 | 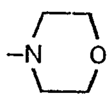 | | H | 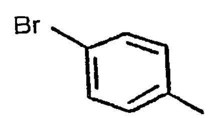 | 139° | 39.70 (40.00 | 3.21 3.36 | 3.37 3.33) |
| 60 | $CH_3$ | $CH_3$ | H | 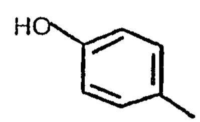 | 85.5–86° | 45.30 (45.69 | 4.19 4.15 | 4.45 4.44) |

| Example No. | $R^1$ | $R^2$ | Y | R | M.P. (°C) | Analysis % or n.m.r. (Theoretical in brackets) C | H | N |
|---|---|---|---|---|---|---|---|---|
| 61 | H | $CH_3$ | H | 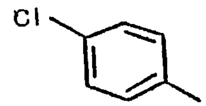 | 110.5–111.5° | 41.30 (41.30 | 3.01 3.15 | 4.32 4.38) |
| 62 | Me | Me | H | 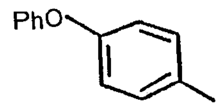 | 61–62° | 55.24 (55.22 | 4.31 4.38 | 3.53 3.58) |
| 63 | Me | Me | H | 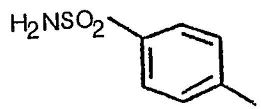 | 124–125° | 38.00 (38.08 | 3.78 3.73 | 7.25 7.40) |
| 64 | Me | Me | H | 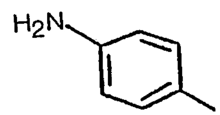 | 132–133° | 48.25 (45.83 | 4.68 4.49 | 9.44 8.91) |
| 65 | Me | –OMe | H | 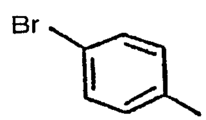 | 72–75° | 36.84 (36.55 | 3.03 3.07 | 3.58 3.55) |

| Example No. | $R^1$ | $R^2$ | Y | R | M.P. (°C) | Analysis % or n.m.r. (Theoretical in brackets) C | H | N |
|---|---|---|---|---|---|---|---|---|
| 66 | H | $-CH_2CF_3$ | H | 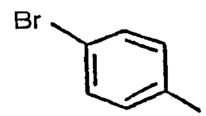 | 120–121° | 33.48 (33.34 | 2.14 2.10 | 3.08 3.24) |
| 67 | H | 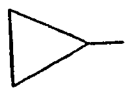 | H | 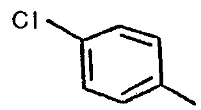 | 81–82° | 44.74 (45.14 | 3.41 3.50 | 4.03 4.05) |
| 68 | Me | Me | H | 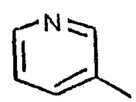 | 75–76° | 43.78 (43.98 | 3.98 4.03 | 9.08 9.33) |
| 69 | Me | Me | H | 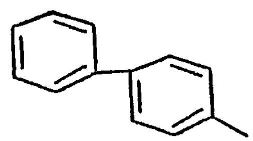 | 117–120° | 58.89 (57.57 | 4.85 4.58 | 3.46 3.73) |

Example 70

Preparation of 5-(4-Bromophenylthio)-N-(t-butoxycarbonylmethyl)-N-methyl-2-thiophenesulphonamide

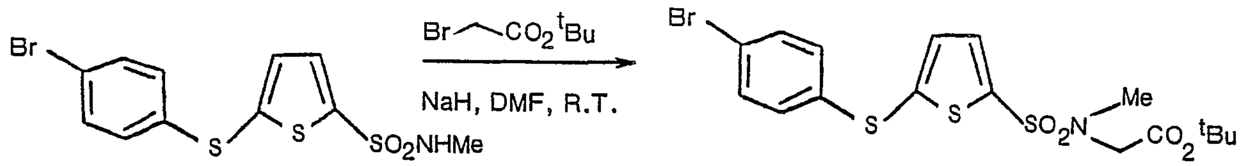


Sodium hydride (0.3 g, 80% dispersion, 0.009 m) was washed with dry petroleum ether (10 ml, 40—60° boiling range, ×3) to remove mineral oil. Dry dimethylformamide (DMF) (20 ml) was added followed by 5-(4-bromophenylthio)-N-methyl-2-thiophenesulphonamide (3 g, 0.0082M). A solution of *t*-butyl $\alpha$-bromoacetate (1.6 g, 0.0082M) in dry dimethylformamide (10 ml) was then added and the mixture was stirred at room temperature for $2\frac{1}{2}$ hours.

The reaction mixture was then poured into water (200 ml) and allowed to stand. The precipitate so obtained was isolated by filtration and dried. Recrystallisation from petroleum ether (60—80° boiling range) gave 5-(4-bromophenylthio)-N-(t-butoxycarbonylmethyl)-N-methyl-2-thiophenesulphonamide (2.4 g, 61.2%) as colourless crystals, m.p. 75—77°.

*Analysis %:*—

| | | | |
|---|---|---|---|
| Found: | C, 42.86; | H, 4.18; | N, 2.73. |
| Required: | C, 42.67; | H, 4.21; | N, 2.93. |

Examples 71—114

The following compounds were prepared by procedures similar to that of Example 70, starting from the appropriate sulfonamide and halide of the formule $R^2$.Hal:—

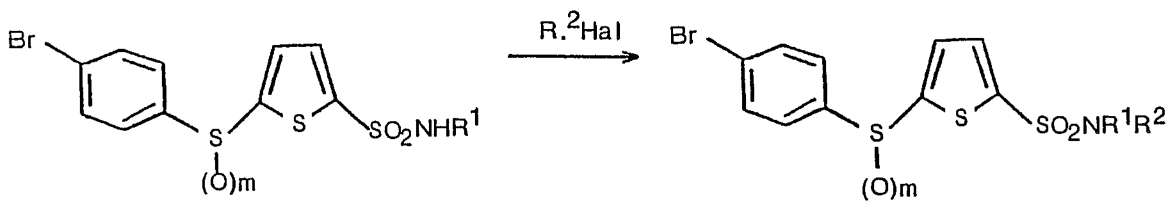


| Example No. | $R^1$ | $R^2$ | Hal | m | M.P. (°C) | Analysis % or n.m.r. (Theoretical in brackets) C | H | N |
|---|---|---|---|---|---|---|---|---|
| 71 (a)* | Me | $-CH_2C{\equiv}CH$ | Br | 0 | 78–80° | 41.54 (41.79 | 2.93 3.00 | 3.52 3.48) |
| 71 (b) | Me | $-CH{=}C{=}CH_2$ | – | 0 | 74–76° | 41.59 (41.79 | 2.91 3.00 | 3.34 3.48) |
| 72 | Me | 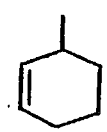 | Br | 0 | 82–84° | 45.64 (45.94 | 4.00 4.08 | 3.19 3.15) |

*In this Example the use of $Br.CH_2C{\equiv}CH$ produced a mixture of products which were separated by chromatography into compounds (a) and (b).

| Example No. | $R^1$ | $R^2$ | Hal | m | M.P. (°C) | Analysis % or n.m.r. (Theoretical in brackets) C | H | N |
|---|---|---|---|---|---|---|---|---|
| 73 | Me | 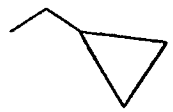 | Br | 0 | 75–76° | 42.79 (43.06 | 3.71 3.85 | 3.08 3.85) |
| 74 | Me | 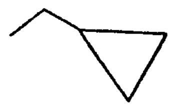 | Br | 2 | 115–116° | 39.71 (40.00 | 3.43 3.58 | 2.95 3.11) |
| 75 | Me | $-CH_2OCO^tBu$ | Cl | 0 | 81–82° | 42.46 (42.67 | 4.26 4.21 | 3.00 2.93) |
| 76 | Me | 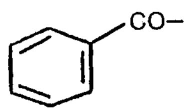 | Cl | 0 | 81–82° | 46.03 (46.15 | 2.99 3.01 | 3.23 2.99) |
| 77 | Me | 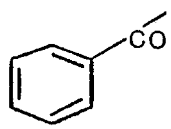 | Cl | 2 | 167° | 43.29 (43.20 | 2.73 2.82 | 3.00 2.80) |
| 78 | Me | $CH_3CO-$ | Cl | 0 | 81–82° | 38.21 (38.42 | 2.94 2.98 | 3.56 3.45) |
| 79 | Me | $PhCH_2CH_2-$ | Br | 2 | 98–99° | 45.60 (45.60 | 3.59 3.63 | 2.85 2.80) |

| Example No. | $R^1$ | $R^2$ | Hal | m | M.P. (°C) | Analysis % or n.m.r. (Theoretical in brackets) C | H | N |
|---|---|---|---|---|---|---|---|---|
| 80 | Me | $ClCH_2CO-$ | Cl | 0 | 71–72° | 35.27 (35.42 | 2.61 2.52 | 3.21 3.18) |
| 81 | Me | 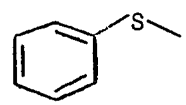 | Cl | 0 | 85–89° | 42.58 (43.21 | 2.97 2.99 | 3.03 2.96) |
| 82 | Me | $-CH_2CHMe_2$ | Br | 0 | 82–3° | 42.60 (42.85 | 4.28 4.32 | 3.36 3.33) |
| 83 | Me | 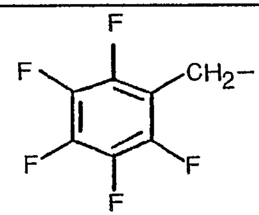 | Br | 0 | 125–6° | 40.04 (39.71 | 1.99 2.04 | 2.70 2.57) |
| 84 | Me | $-(CH_2)_2OPh$ | Br | 0 | 95–6° | 47.51 (47.10 | 3.78 3.75 | 2.83 2.89) |
| 85 | Me | $-(CH_2)_3OPh$ | Br | 0 | 68–9° | 47.92 (48.19 | 3.96 4.04 | 2.71 2.81) |
| 86 | Me | $-(CH_2)_4OPh$ | Br | 0 | 77–8° | 49.52 (49.21 | 4.26 4.33 | 2.84 2.73) |
| 87 | Me | 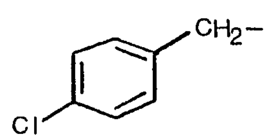 | Br | 0 | 108–9° | 44.41 (44.22 | 3.06 3.09 | 2.61 2.87) |

| Example No. | $R^1$ | $R^2$ | Hal | m | M.P. (°C) | Analysis % or n.m.r. (Theoretical in brackets) C | H | N |
|---|---|---|---|---|---|---|---|---|
| 88 | Me | 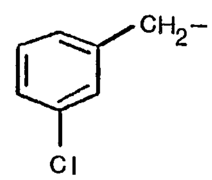 | Br | 0 | 79° | 44.62 (44.22 | 3.00 3.09 | 2.86 2.87) |
| 89 | Me | 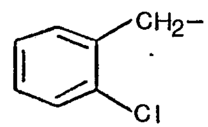 | Br | 0 | 76–7° | 44.32 (44.22 | 3.01 3.09 | 2.64 2.87) |
| 90 | Me | 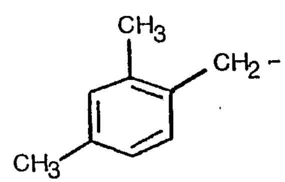 | Cl | 0 | 53° | 49.78 (49.78 | 4.14 4.18 | 2.89 2.90) |
| 91 | Me | 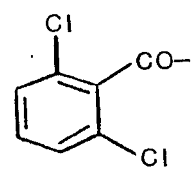 | Cl | 0 | Oil | 8.0–7.0 (m with s at 7.3,9H,Ar–H), 3.4–3.0 (m,3H,NMe) | | |
| 92 | Me | $Me_2NSO_2-$ | Cl | 0 | 85° | 33.32 (33.12 | 3.25 3.21 | 5.85 5.94) |

| Example No. | $R^1$ | $R^2$ | Hal | m | M.P. (°C) | Analysis % or n.m.r. (Theoretical in brackets) C | H | N |
|---|---|---|---|---|---|---|---|---|
| 93 | Me | 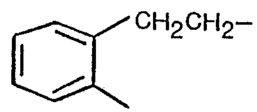 | Br | 0 | 72–4° | 48.75<br>(48.71 | 3.77<br>3.87 | 2.94<br>2.99) |
| 94 | Me | 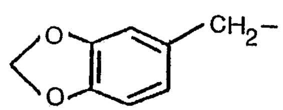 | Br | 0 | 101–3° | 46.05<br>(45.78 | 3.31<br>3.24 | 2.80<br>2.81) |
| 95 | Me | $CH_3OCH_2CH_2OCH_2-$ | Cl | 0 | 75–6° | 39.80<br>(39.82 | 3.89<br>4.01 | 3.06<br>3.10) |
| 96 | Me | $CH_3OCH_2CH_2OCH_2-$ | Cl | 2 | 86–8° | 37.28<br>(37.19 | 3.68<br>3.75 | 3.03<br>2.89) |

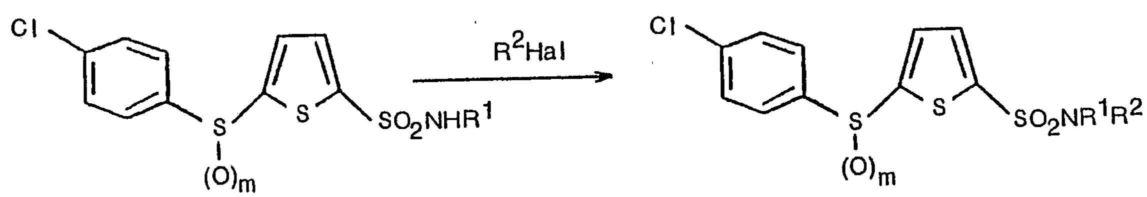


| Example No. | $R^1$ | $R^2$ | Hal | m | M.P. (°C) | Analysis % or n.m.r. (Theoretical in brackets) C | H | N |
|---|---|---|---|---|---|---|---|---|
| 97 | Me | $CH_3CO-$ | Cl | 0 | 66° | 43.14<br>(43.14 | 3.31<br>3.34 | 3.87<br>3.87) |
| 98 | Me | $(CH_3)_2CHCO-$ | Cl | 0 | 65° | 46.20<br>(46.20 | 4.07<br>4.14 | 3.60<br>3.59) |
| 99 | Me | $CH_3(CH_2)_2CO-$ | Cl | 0 | Oil | 7.45(d,J 5Hz,1H), 7.2(s,4H), 7.05(d,J 5Hz,1H), 3.2(s,3H), 2.06(t,J 8Hz,2H), 1.8–1.2 (m,2H), 1.1(t,J 7Hz,3H) | | |
| 100 | Me | $CH_3(CH_2)_{14}CO-$ | Cl | 0 | Oil | 7.5(d,J 5Hz,1H), 7.3(s,4H), 7.1(d,J 5Hz,1H), 3.3(s,3H), 2.7(m,2H), 1.8–0.8(m with broad s at 1.2,29H) | | |
| 101 | Me | $(CH_3)_3C.CO-$ | Cl | 0 | 77–8° | 47.67<br>(47.57 | 4.41<br>4.49 | 3.44<br>3.47) |
| 102 | Me | 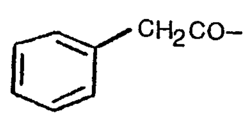 | Cl | 0 | 73–5° | 52.23<br>(52.10 | 3.56<br>3.68 | 3.18<br>3.20) |

| Example No. | $R^1$ | $R^2$ | Hal | m | M.P. (°C) | Analysis % or n.m.r. (Theoretical in brackets) C | H | N |
|---|---|---|---|---|---|---|---|---|
| 103 | Me | 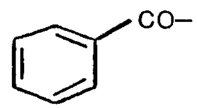 | Cl | 0 | 131–2° | 50.81 (50.99 | 3.29 3.33 | 3.21 3.30) |
| 104 | Me | 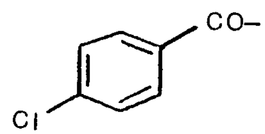 | Cl | 0 | 85–6° | 47.89 (47.16 | 2.76 2.86 | 2.81 3.06) |
| 105 | Me | 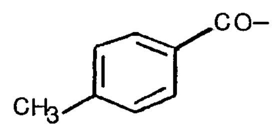 | Cl | 0 | 112–4° | 52.02 (52,10 | 3.56 3.68 | 3.17 3.20) |
| 106 | Me | 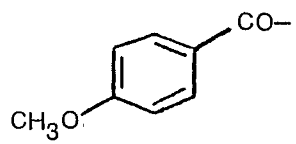 | Cl | 0 | 109–110° | 50.27 (50.26 | 3.44 3.55 | 2.98 3.09) |
| 107 | Me | 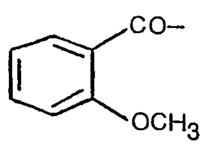 | Cl | 0 | Oil | 7.65(d,J 5Hz,1H), 7.3(s,4H), 7.3–6.8(m,5H), 3.7(s,3H), 3.2(s,3H) | | |

| Example No. | $R^1$ | $R^2$ | Hal | m | M.P. (°C) | Analysis % or n.m.r. (Theoretical in brackets) C | H | N |
|---|---|---|---|---|---|---|---|---|
| 108 | Me | 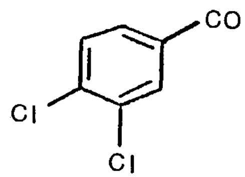 | Cl | 0 | 104–6° | 44.17 (43.86 | 2.46 2.45 | 2.82 2.84) |
| 109 | Me | 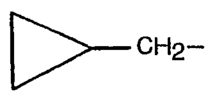 | Br | 0 | 72–3° | 48.22 (48.18 | 4.31 4.31 | 3.79 3.79) |

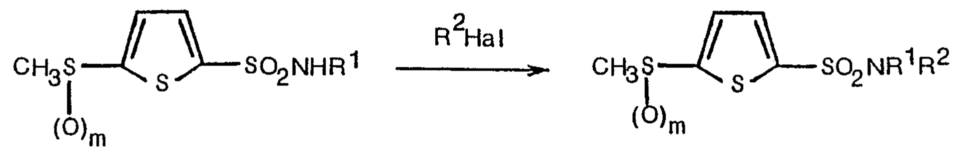


| Example No. | $R^1$ | $R^2$ | Hal | m | M.P. (°C) | Analysis % or n.m.r. (Theoretical in brackets) C | H | N |
|---|---|---|---|---|---|---|---|---|
| 110 | Me | $-CH_2CH{=}CH_2$ | Br | 0 | Oil | 40.94 (41.04 | 4.86 4.97 | 5.19 5.32) |
| 111 | Me | 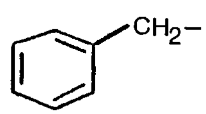 | Br | 0 | 102–104° | 49.12 (49.81 | 4.68 4.82 | 4.43 4.47) |
| 112 | Me | 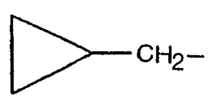 | Br | 0 | Oil | 42.84 (43.29 | 5.41 5.45 | 4.96 5.05) |
| 113 | Me | $CH_3CO-$ | Cl | 0 | Oil | 34.89 (36.21 | 3.98 4.18 | 5.54 5.28) |
| 114 | Me | 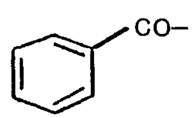 | Cl | 0 | Oil | 45.40 (47.68 | 3.98 4.00 | 4.33 4.28) |

Examples 115 to 118

The following compounds were prepared by procedures similar to that of Example 70, starting from the appropriate sulphonamide and methyl iodide:—

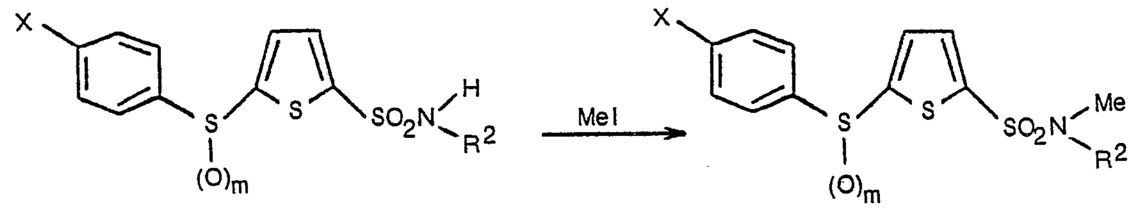


| Example No. | X | m | $R^2$ | M.P. (°C) | Analysis % (Theoretical in brackets) C | H | N |
|---|---|---|---|---|---|---|---|
| 115 | Br | 0 | 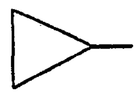 | 71–72° | 41.60<br>(41.58 | 3.45<br>3.49 | 3.53<br>3.46) |
| 116 | Br | 2 | 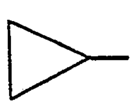 | 142–3° | 38.63<br>(38.53 | 3.25<br>3.23 | 3.01<br>3.21) |
| 117 | Cl | 0 | 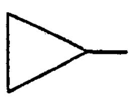 | 88–90° | 46.34<br>(46.72 | 3.82<br>3.92 | 3.88<br>3.89) |
| 118 | Br | 0 | $CF_3CH_2-$ | 100–102° | 35.30<br>(34.98 | 2.51<br>2.48 | 3.34<br>3.14) |

Example 119

Preparation of 5-(4-Nitrophenylthio)-N,N-dimethyl-2-thiophenesulphonamide

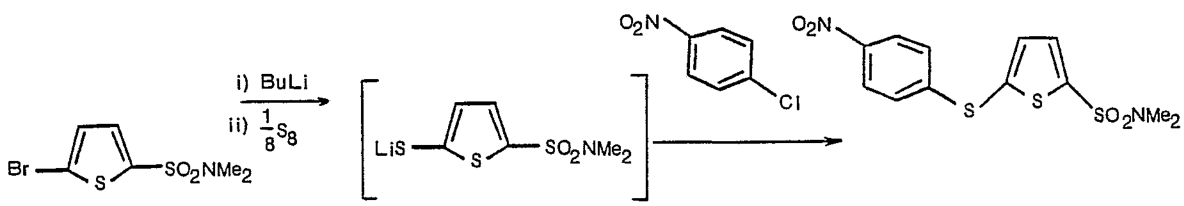


A stirred solution of 5-bromo-N,N-dimethyl-2-thiophenesulphonamide (2.7 g, 0.01 m) in anhydrous ether (50 ml) was cooled to −70° under an atmosphere of dry nitrogen. *n*-Butyl lithium (6.3 ml, 1.6 m solution in hexane, 0.01M) was added at such a rate that the internal temperature was maintained below −60°. The resulting white suspension was stirred for 15 minutes and then sulphur (0.32 g, 0.01M) was added. The mixture was stirred at −70° for 1 hour and then allowed to warm to room temperature. The solvent was removed *in vacuo* and the yellow solid which remained was taken up in anhydrous dimethylformamide (20 ml). To this, a solution of 1-chloro-4-nitrobenzene (1.57 g, 0.01M) was added and the mixture was stirred at room temperature for 16 hours.

The solution was poured into saturated sodium chloride solution (100 ml) and extracted with ether (100 ml, ×2). The combined organic layers were washed with water (100 ml, ×2), dilute hydrochloric acid (100 ml, 5%) and water (100 ml). After drying ($CaCl_2$) the ether was evaporated to leave partially crystalline material.

This was triturated with petroleum ether (b.p. 40—60°) to give a brown solid which was recrystallised from chloroform/petroleum ether (b.p. 40—60°) to give 5-(4-nitrophenylthio)-N,N-dimethyl-2-thiophenesulphonamide (0.43 g, 12.5%), m.p. 95—103°.

*Analysis %:—*

| | | | |
|---|---|---|---|
| Found: | C, 41.67; | H, 3.56; | N, 8.07. |
| Required: | C, 41.84; | H, 3.51; | N, 8.13. |

Examples 120—128

The following compounds were prepared by procedures similar to that of Example 119, starting from 5-bromo-N,N-dimethyl-2-thiophenesulphonamide, *n*-butyl lithium, sulphur and the appropriate halide of the formula R.Hal:

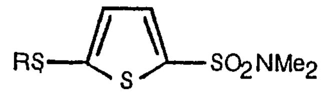

| Example No. | R | Hal | M.P. (°C) | Analysis % or n.m.r. (Theoretical in brackets) C | H | N |
|---|---|---|---|---|---|---|
| 120 | $CH_2=CH.CH_2-$ | Br | oil | 2.75 (s, 6H), 3.55 (d, J6Hz, 2H), 5.0 (d, J6Hz, 1H), 5.2 (s, 1H), 5.6 – 6.3 (m, 1H), 7.1 (d, J5Hz, 1H), 7.4 (d, J5Hz, 1H) | | |
| 121 | $PhCH_2CH_2-$ | Br | oil | 2.76 (s, 6H), 2.7 – 3.25 (m, 4H), 2.02 (d, J 5Hz, 1H), 7.21 (s, (broad), 5H) 7.25 (d, J 5Hz, 1H) | | |
| 122 | $CH_3OCH_2-$ | Cl | oil | 36.51<br>(35.93 | 4.98<br>4.90 | 5.08<br>5.24) |
| 123 | $CH_2=C=CH-$ | Br* | oil | 2.76 (s, 6H), 4.95 (d, J6Hz, 2H), 5.9 (t, J6Hz, 1H), 7.05 (d, J5Hz, 1H), 7.35 (d, J5Hz, 1H) | | |
| 124 | *n*-Bu– | Br | oil | 0.7 – 2.0 (m, 7H), 2.6 – 3.2 (m, 2H) 2.75 (s, 6H), 7.05 (d, J 5Hz, 1H), 7.37 (d, J 5Hz, 1H) | | |
| 125 | 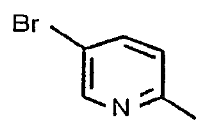 | Br | 108–109.5° | 35.32<br>(34.83 | 2.96<br>2.92 | 7.51<br>7.39) |

* From propargyl bromide.

| Example No. | R | Hal | M.P. (°C) | Analysis % or n.m.r. (Theoretical in brackets) C | H | N |
|---|---|---|---|---|---|---|
| 126 | 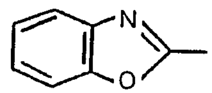 | Cl | 70–120° (dec) | 45.24 (45.86 | 3.70 3.55 | 7.97 8.23) |
| 127 | 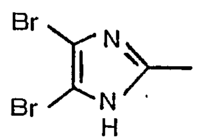 | Br | 54–56° | 24.80 (24.17 | 2.05 2.03 | 9.24 9.40) |
| 128 | 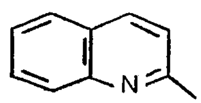 * | Cl | 145–150° | 46.40 (46.56 | 3.77 3.91 | 7.13 7.24) |

*Analysed as HCl salt

Example 129

Preparation of 4-Phenylsulphinyl-N,N-Dimethyl-2-thiophenesulphonamide

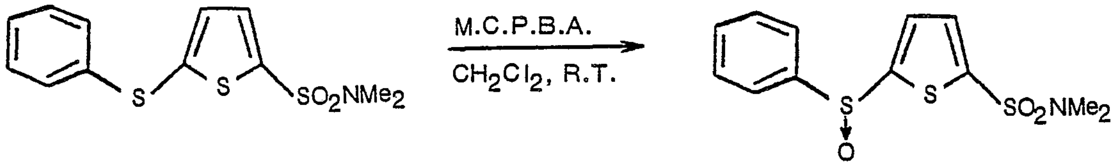


To a stirred solution of 5-phenylthio-N,N-dimethyl-2-thiophenesulphonamide (2.1 g, 0.007 m) in dichloromethane (20 ml) was added *m*-chloroperbenzoic acid (MCPBA) (1.3 g, 0.0075 m) and the mixture was heated under reflux for two hours. The cooled solution was washed with saturated sodium carbonate solution (20 ml, ×2) and dried ($MgSO_4$). The solvent was removed *in vacuo* and the residue taken up in ethyl acetate (20 ml). This was passed down a short column of silica (20 g, "Whatmans" S13 TLC) using ethyl acetate (200 ml) as eluant. The eluate was evaporated to give 5-phenylsulphinyl-N,N-dimethyl-2-thiophenesulphonamide (0.84 g, 38%), m.p. 87—88°.

*Analysis %:—*

| | | | |
|---|---|---|---|
| Found: | C, 45.50; | H, 4.12; | N, 4.47 |
| Required: | C, 45.69; | H, 4.15; | N, 4.44 |

Examples 130—135

The following compounds were prepared by procedures similar to that of Example 129, starting from the appropriate phenylthiosulphonamide and *m*-chloroperbenzoic acid:

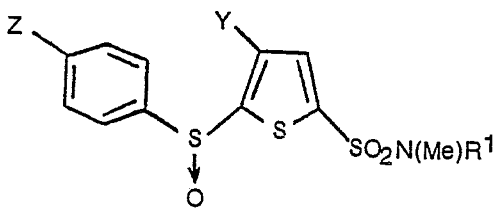

| Example No. | Z | $R^1$ | Y | m.p. (°C) | Analysis (%) (Theoretical in brackets) C | H | N |
|---|---|---|---|---|---|---|---|
| 130 | F | Me | H | 85–88 | 43.23 (43.23 | 3.52 3.60 | 4.16 4.20) |
| 131 | Cl | Me | H | 93–4 | 40.93 (41.19 | 3.42 3.45 | 4.93 4.00) |
| 132 | $CH_3O$ | Me | H | 96–99 | 45.09 (45.19 | 4.33 4.37 | 4.05 4.05) |
| 133 | $CH_3$ | Me | Br | 182–3 | 38.44 (38.24 | 3.47 3.46 | 3.41 3.43) |
| 134 | Br | Me | H | 105–7 | 36.77 (36.55 | 2.99 3.07 | 3.82 3.55) |
| 135 | Cl | H | H | 142–5 | 39.34 (39.39 | 3.00 2.95 | 4.17 4.34) |

Example 136

Preparation of 5-Phenylsulphonyl-N,N-Dimethyl-2-Thiophenesulphonamide

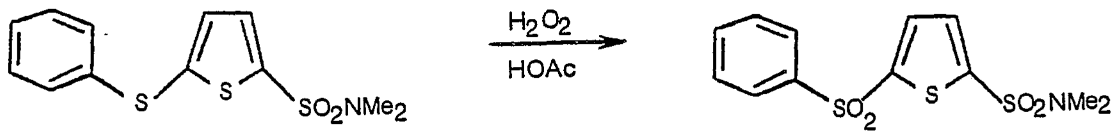


To a stirred solution of 5-phenylthio-N,N-dimethyl-2-thiophene-sulphonamide (2.3 g, 0.0077 m) in glacial acetic acid (25 ml) was added hydrogen peroxide solution (3.6 mm of 100 volume, 0.03 m) and the mixture was heated under reflux for two hours. The mixture was poured into water (100 ml) and the resulting oil was separated by decantation. On standing the oil crystallised.

Recrystallisation from ethyl acetate/petroleum ether (40—60° boiling range) gave 5-phenylsulphonyl-N,N-dimethyl-2-thiophenesulphonamide (2.06 g, 81%), m.p. 91—93°.

*Analysis %:—*

| | | | |
|---|---|---|---|
| Found: | C, 43.54; | H, 4.02; | N, 4.26 |
| Required: | C, 43.48; | H, 3.95; | N, 4.22 |

Examples 137—185

The following compounds were prepared by procedures similar to that of Example 136, starting from the appropriate thiosulphonamide, hydrogen peroxide and glacial acetic acid:—

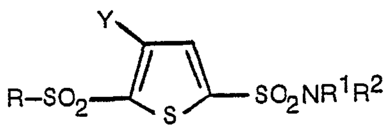

| Example No. | $R^1$ | $R^2$ | Y | R | m.p. (°C) | Analysis (%) (Theoretical in Brackets) C | H | N |
|---|---|---|---|---|---|---|---|---|
| 137 | Me | Me | H | 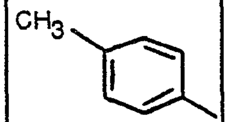 | [illegible]5–138 | 44.79 (45.20 | 4.40 4.38 | 3.83 4.05) |
| 138 | Me | Me | H | 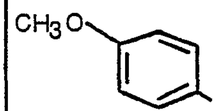 | 141–3 | 43.48 (43.19 | 4.17 4.18 | 3.71 3.87) |
| 139 | Me | Me | H | 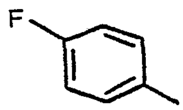 | 105–110 | 41.01 (41.25 | 3.36 3.44 | 3.95 4.01) |
| 140 | Me | Me | H | 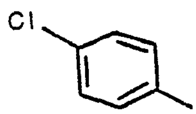 | 166–168 | 39.43 (39.39 | 3.24 3.30 | 3.78 3.83) |
| 141 | Me | Me | H | 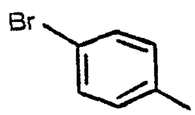 | 177–178 | 35.45 (35.12 | 3.07 2.94 | 3.68 3.41) |
| 142 | Me | Me | H | 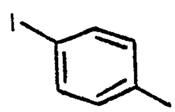 | 194–196 | 31.95 (31.51 | 2.70 2.64 | 3.27 3.06) |
| 143 | Me | Me | H | 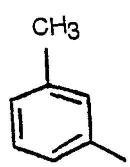 | 131.5 | 44.89 (45.20 | 4.29 4.38 | 4.01 4.05) |
| 144 | Me | Me | H | 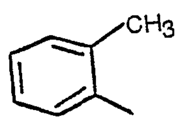 | 102–103 | 44.85 (45.20 | 4.31 4.38 | 4.10 4.05) |
| 145 | Me | Me | H | 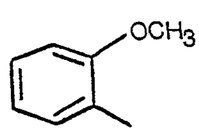 | 133 | 43.00 (43.20 | 4.14 4.18 | 3.94 3.88) |

| Example No. | $R^1$ | $R^2$ | Y | R | m.p. (°C) | Analysis (%) (Theoretical in Brackets) C | H | N |
|---|---|---|---|---|---|---|---|---|
| 146 | Me | Me | H | 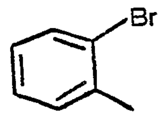 | 129–130 | 34.80 (35.12 | 2.82 2.93 | 3.37 3.41) |
| 147 | Me | Me | H | 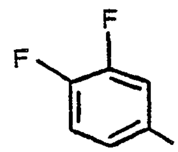 | 157 | 39.43 (39.23 | 2.98 3.02 | 3.80 3.81) |
| 148 | Me | Me | H | 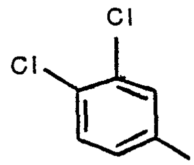 | 173–5 | 36.11 (36.00 | 2.81 2.77 | 3.84 3.49) |
| 149 | Me | Me | H | 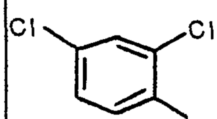 | 144–5 | 36.05 (36.00 | 2.68 2:75 | 3.49 3.50) |
| 150 | Me | Me | Br | 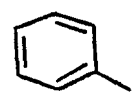 | 148–150 | 35.25 (35.12 | 2.91 2.95 | 3.22 3.41) |
| 151 | Me | Me | Br | 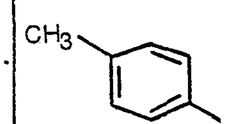 | 163–165 | 37.04 (36.37 | 3.38 3.33 | 3.28 3.30) |
| 152 | Me | Me | Br | 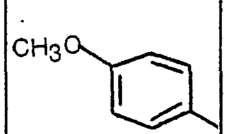 | 166–8 | 35.41 (35.45 | 3.24 3.20 | 3.08 3.18) |
| 153 | Me | Me | Br | 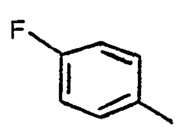 | 141–3 | 33.46 (33.65 | 2.71 2.59 | 3.16 3.27) |
| 154 | Me | Me | Br | 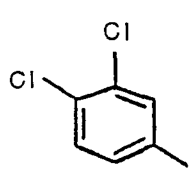 | 167–9 | 30.52 (30.07 | 2.15 2.10 | 2.98 2.92) |

| Example No. | $R^1$ | $R^2$ | Y | R | m.p. (°C) | Analysis (%) (Theoretical in Brackets) C | H | N |
|---|---|---|---|---|---|---|---|---|
| 155 | Me | Me | Me | 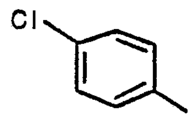 | 121–122 | 41.26 (41.10 | 3.68 3.71 | 3.62 3.69) |
| 156 | H | Me | H | 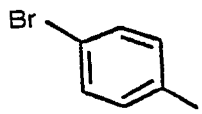 | 127–9 | 33.48 (33.33 | 2.62 2.54 | 3.64 3.53) |
| 157 | H | Et | H | 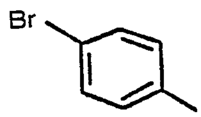 | 127–129 | 35.40 (35.12 | 2.94 2.95 | 3.53 3.41) |
| 158 | H | iPr | H | 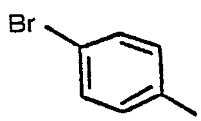 | 80–82 | 36.32 (36.79 | 3.22 3.33 | 3.16 3.30) |
| 159 | Me | Et | H | 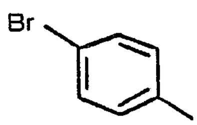 | 116 | 36.85 (36.79 | 3.36 3.33 | 3.65 3.30) |
| 160 | Me | iPr | H | 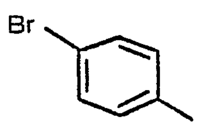 | 98 | 38.44 (38.36 | 3.58 3.68 | 3.33 3.20) |
| 161 | Me | tBu | H | 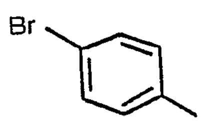 | 108–109 | 39.97 (39.82 | 4.13 4.01 | 3.10 3.10) |
| 162 | Me | allyl | H | 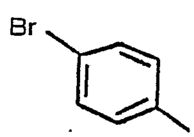 | 97 | 38.77 (38.53 | 3.14 3.23 | 3.27 3.21) |
| 163 | Me | Propargyl | H | 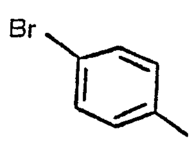 | 88–90 | 38.67 (38.71 | 2.77 2.78 | 3.11 3.22) |

| Example No. | $R^1$ | $R^2$ | Y | R | m.p. (°C) | Analysis (%) (Theoretical in Brackets) C | H | N |
|---|---|---|---|---|---|---|---|---|
| 164 | Me | Cyclopentyl | H | 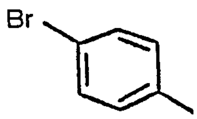 | 160–161 | 41.18<br>(41.38 | 3.84<br>3.91 | 3.02<br>3.02) |
| 165 | Me | Cyclohexyl | H | 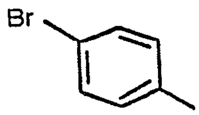 | 150–152 | 42.35<br>(42.67 | 4.11<br>4.21 | 2.86<br>2.93) |
| 166 | Me | Phenyl | H | 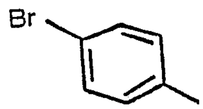 | 106–107 | 43.11<br>(43.22 | 2.93<br>2.99 | 3.03<br>2.97) |
| 167 | Me | Benzyl | H | 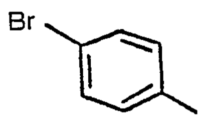 | 155 | 44.63<br>(44.44 | 3.34<br>3.32 | 2.80<br>2.88) |
| 168 | Et | Et | H | 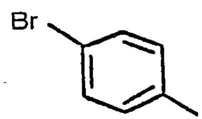 | 124–125 | 38.03<br>(38.36 | 3.63<br>3.68 | 3.35<br>3.20) |
| 169 | 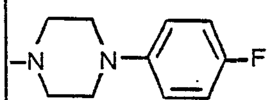 | | H | 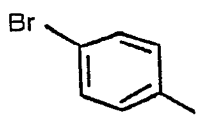 | 174–176 (Dec.) | 42.87<br>(42.63 | 3.31<br>3.58 | 4.75<br>4.97) * |
| 170 | 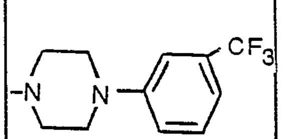 | | H | 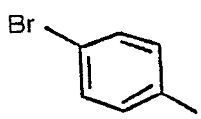 | 188–192 | 42.13<br>(42.36 | 3.73<br>3.05 | 4.19<br>4.70) * |
| 171 | 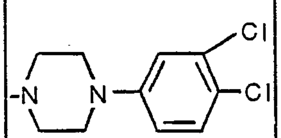 | | H | 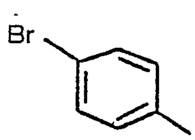 | 160–165 (Dec.) | 38.96<br>(39.10 | 2.81<br>3.12 | 4.43<br>4.56) |
| 172 | Me | Me | H | Me | 118–119 | 31.38<br>(31.21 | 4.02<br>4.12 | 4.92<br>5.20) |
| 173 | Me | Me | Me | Me | 155–156 | 33.69<br>(33.90 | 4.50<br>4.62 | 4.90<br>4.94) |

*analysed as monohydrates

| Example No. | $R^1$ | $R^2$ | Y | R | m.p. (°C) | Analysis (%) (Theoretical in brackets) C | H | N |
|---|---|---|---|---|---|---|---|---|
| 174 | Me | Me | H | 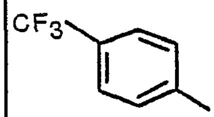 | 169–171 | 39.29 (39.09 | 3.00 3.03 | 3.45 3.50) |
| 175 | H | Cyclopropyl | H | 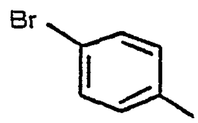 | 149 | 36.86 (36.97 | 2.56 2.86 | 3.30 3.32) |
| 176 | Me | $-CH_2CO_2tBu$ | H | 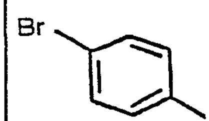 | 130–132 | 40.42 (40.00 | 3.92 3.95 | 2.58 2.74) |
| 177 | 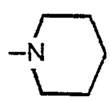 | | H | 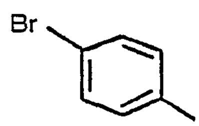 | 133–134 | 39.79 (40.00 | 3.88 3.58 | 3.13 3.11) |
| 178 | 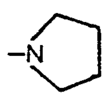 | | H | 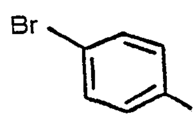 | 132–133 | 38.36 (38.53 | 3.09 3.23 | 3.09 3.21) |
| 179 | 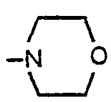 | | H | 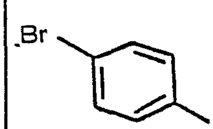 | 110–111 | 37.17 (37.17 | 3.07 3.12 | 3.34 3.10) |
| 180 | H | Me | H | 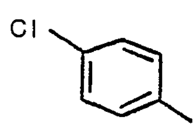 | 126–128 | 37.67 (37.55 | 2.84 2.86 | 3.91 3.98 |
| 181 | H | cyclopropyl | H | 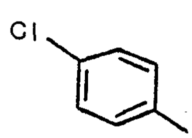 | 144–146 | 41.25 (41.32 | 3.13 3.20 | 3.64 3.71) |
| 182 | Me | Me | H | 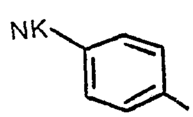 | 174–175 | 43.49 (43.80 | 3.30 3.39 | 7.85 7.86) |

| Example No. | $R^1$ | $R^2$ | Y | R | m.p. (°C) | Analysis (%) (Theoretical in Brackets) C H N |
|---|---|---|---|---|---|---|
| 183 | Me | Me | H | 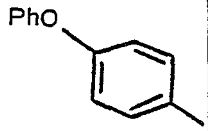 | 129—131 | 50.64 3.99 3.32<br>(51.04 4.05 3.31) |
| 184 | Me | 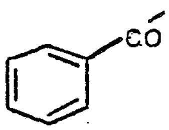 | H | 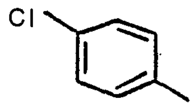 | 156—158° | 47.28 3.07 3.27<br>(47.41 3.09 3.07) |
| 185 | Me | $CH_3CO-$ | H | 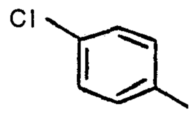 | 136 | 39.23 2.98 3.65<br>(39.66 3.07 3.55) |

Example 186

Preparation of 5-(4-Bromophenoxy)-N,N-dimethyl-2-thiophenesulphonamide

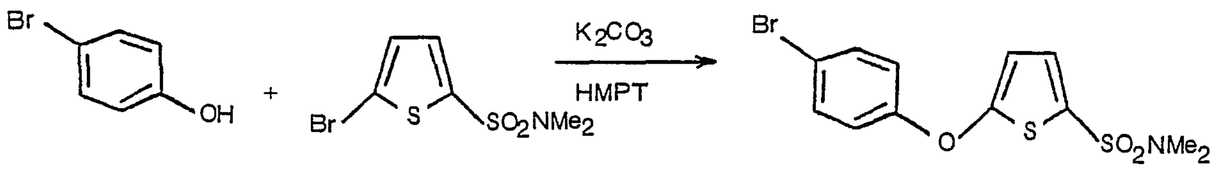


A stirred suspension of anhydrous potassium carbonate (3.78 g, 0.03 m) in a solution of *p*-bromophenol (3.46 g, 0.02 m) and 5-bromo-N,N-dimethyl-2-thiophenesulphonamide (5.4 g, 0.02 m) in hexamethylphosphoric triamide (HMPT) (30 ml) was heated at 120° under an atmosphere of dry nitrogen for 24 hours. The cooled reaction mixture was poured into water (300 ml) and extracted with ethyl acetate (30 ml, x4). The combined organic layers were washed with sodium hydroxide solution (30 ml, 5%, x2), water (30 ml, x5), saturated sodium chloride solution (30 ml) and then dried ($MgSO_4$). The solvent was removed *in vacuo* leaving a pale brown oil. The oil was extracted with hot petrol (40°—60° boiling range, 50 ml, x4). The combined petrol extracts were set aside to cool. A white solid was collected by filtration and recrystallised from ethyl acetate/petroleum ether (60°—80° boiling range) giving 5-(4-bromophenoxy)-N,N-dimethyl-2-thiophenesulphonamide as colourless crystals (1.7 g, 23%), m.p. 55°C.

*Analysis %:—*

| | | | |
|---|---|---|---|
| Found: | C, 39.46; | H, 3.28; | N, 3.90 |
| Required: | C, 39.78; | H, 3.34; | N, 3.87 |

Example 187

Preparation of 5-(4-Chlorophenylthio)-4-methoxy-N,N-dimethyl-2-thiophenesulphonamide

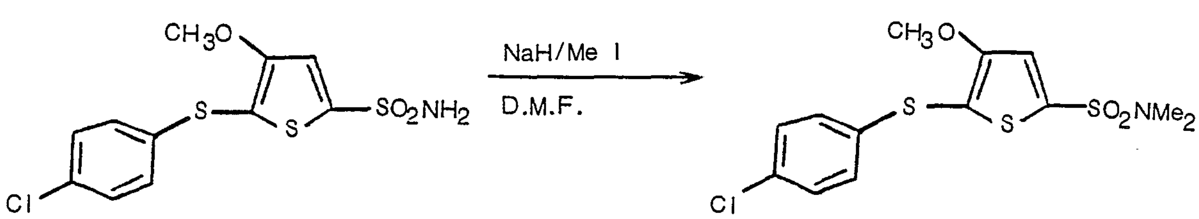


The mineral oil was removed from sodium hydride (60 mg, 80%, 0.002 m) by washing with dry

petroleum ether (5 ml, ×2). The petroleum ether was removed and replaced by dry dimethylformamide (DMF) (5 ml) and the suspension was cooled to 0°. A solution of 5-(4-chlorophenylthio)-4-methoxy-2-thiophenesulphonamide (0.270 g, 0.0008 m) (see Example 60 of British Patent No. 1459571) in dry dimethylformamide (5 ml) was added dropwise over a period of five minutes. Methyl iodide (0.25 g, 0.0018 m) was then added and the mixture stirred for 0.5 hours during which time it was allowed to attain room temperature. The reaction mixture was poured into water (50 ml) and extracted with dichloromethane (10 ml ×3). The combined organic layers were washed with water (30 ml ×2), saturated sodium chloride solution (30 ml) and then dried ($MgSO_4$). The solvent was removed *in vacuo* to leave an oil which crystallised on standing. The crude product was recrystallised from ethyl acetate/petroleum ether (60—80° boiling range) to give 5-(4-chlorophenylthio)-4-methoxy-N,N-dimethyl-2-thiophenesulphonamide (220 mg, 75%) as colourless crystals, m.p. 112—114°C.

*Analysis %:—*

| | | | |
|---|---|---|---|
| Found: | C, 43.17; | H, 3.89; | N, 4.03 |
| Required: | C, 42.90; | H, 3.87; | N, 3.85 |

Examples 188—204

The following compounds were prepared similarly to Example 187, starting from the appropriate sulphonamide, sodium hydride and methyl iodide:—

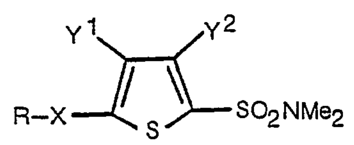

| Example No. | $Y^1$ | R | $Y^2$ | X | m.p. (°C) | Analysis (%) (Theoretical in Brackets) C | H | N |
|---|---|---|---|---|---|---|---|---|
| 188 | MeO | 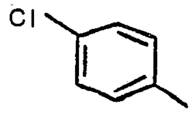 | H | $SO_2$ | 178–180 | 39.49 (39.44 | 3.54 3.56 | 3.62 3.54) |
| 189* | H | 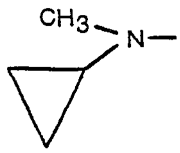 | H | $SO_2$ | 112–114 | 36.85 (37.02 | 4.99 4.97 | 8.88 8.64) |
| 190 | H | 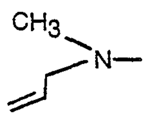 | H | $SO_2$ | 92–93 | 37.42 (37.02 | 4.86 4.97 | 8.56 8.64) |
| 191 | H | 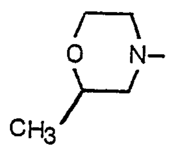 | H | $SO_2$ | 120–121 | 37.25 (37.27 | 5.26 5.12 | 7.94 7.90) |
| 192 | H | 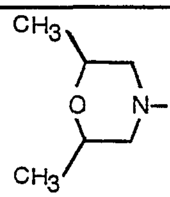 | H | $SO_2$ | 150–152 | 39.39 (39.11 | 5.61 5.47 | 7.35 7.60) |
| 193 | H | 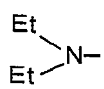 | H | $SO_2$ | 88–90 | 36.79 (36.79 | 5.58 5.55 | 8.23 8.58) |
| 194* | H | 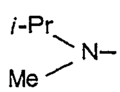 | H | $SO_2$ | 105–107 | 36.81 (36.79 | 5.67 5.55 | 8.56 8.58) |
| 195 | H | 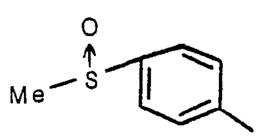 | H | S | 88–91 | 43.43 (43.19 | 4.17 4.18 | 3.81 3.87) |
| 196 | H | 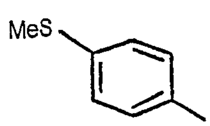 | H | S | 86–88 | 44.92 (45.19 | 4.34 4.38 | 3.96 4.05) |

| Example No. | $Y^1$ | R | $Y^2$ | X | m.p. (°C) | Analysis (%) (Theoretical in Brackets) C | H | N |
|---|---|---|---|---|---|---|---|---|
| 197 | H | 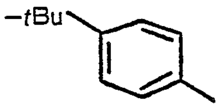 | H | $SO_2$ | 199–200 | 49.25 (49.59 | 5.41 5.46 | 3.54 3.61) |
| 198 | H | 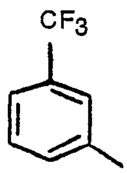 | H | $SO_2$ | 145–146 | 39.24 (39.09 | 2.89 3.03 | 3.54 3.51) |
| 199 | H | 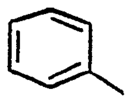 | Br | $SO_2$ | 126.5–128 | 35.48 (35.12 | 2.91 2.95 | 3.17 3.41) |
| 200 | H | 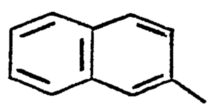 | H | $SO_2$ | 148–149 | 49.92 (50.37 | 3.92 3.96 | 3.73 3.67) |
| 201 | H | 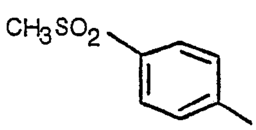 | H | S | 136 | 41.25 (41.36 | 3.97 4.01 | 3.74 3.71) |
| 202 | H | 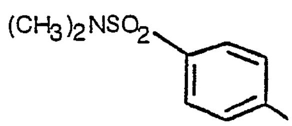 | H | S | 142–143 | 41.27 (41.36 | 4.37 4.46 | 6.62 6.89) |
| 203 | H | 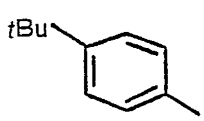 | H | S | 55–56 | 54.19 (54.05 | 5.97 5.95 | 3.57 3.94) |
| 204 | H | 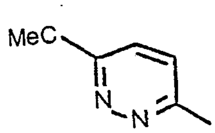 | H | S | 108–110 | 38.96 (39.86 | 3.96 3.95 | 12.03 12.68) |

*These products were prepared by trimethylation using sulphonamide starting materials in which R was 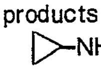NH– and *i*-PrNH– respectively.

Example 205

Preparation of 5-(p-bromophenylthio)-3-trimethylsilyl-N,N-dimethyl-2-thiophenesulphonamide

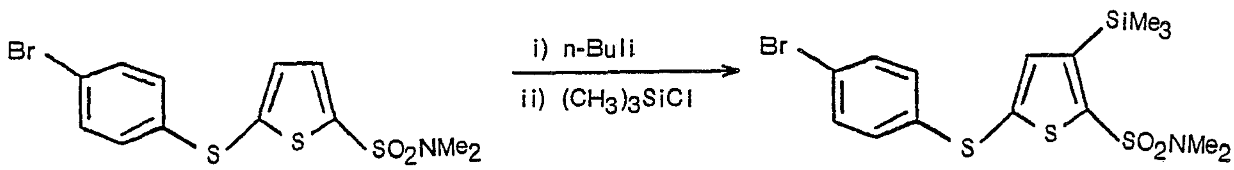


A stirred solution of 5-(*p*-bromophenylthio)-N,N-dimethyl-2-thiophenesulphonamide (3.78 g, 0.01 M) in anhydrous ether (80 ml) was cooled to −70° under an atmosphere of dry nitrogen. *n*-Butyl lithium (6.6 ml), 1.6 molar solution in hexane, 0.0105 M) was added at such a rate that the internal temperature was maintained below −60°. The resulting mixture was stirred for 15 minutes. A solution of trimethylsilylchloride (1.4 ml, 0.011 M) in anhydrous ether (20 ml) was then added. The cooling bath was removed and the mixture allowed to warm to room temperature over a period of 2½ hours. The reaction mixture was filtered and evaporated leaving a gum which was crystallized twice from 130-octane to give the title compound 2.1 g, 77%, m.p. 78—80°.

*Analysis %:—*

| | | | |
|---|---|---|---|
| Found: | C, 40.27; | H, 4.59; | N, 3.22. |
| Required: | C, 39.99; | H, 4.47; | N, 3.11. |

Examples 206—210

The following compounds were prepared by procedures similar to that of Example 205 starting from the appropriate sulphonamide and the stated electrophile. In the cases marked (*) the filtration step in the Example 205 was replaced by extraction with 5% aqueous sodium hydroxide solution and drying ($MgSO_4$).

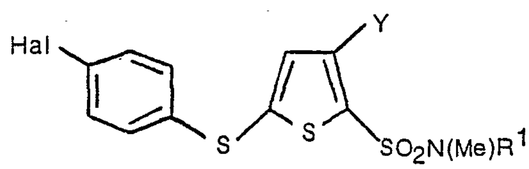

| Example No. | Hal | $R^1$ | Y | electrophile used | m.p. (°C) | Analysis % or n.m.r. (Theoretical in brackets) C | H | N |
|---|---|---|---|---|---|---|---|---|
| 206* | Cl. | Me | 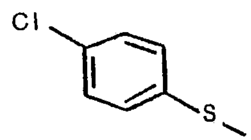 | 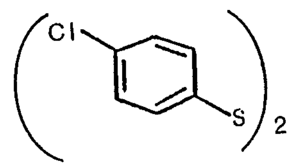 | 113–115 | 45.22 (45.37 | 3.04 3.17 | 3.01 2.94) |
| 207* | Cl | Me | MeS– | $(MeS)_2$ | 134–135 | 41.61 (41.09 | 3.63 3.71 | 3.77 3.69) |
| 208 | Cl | Me | Me– | MeI | 59–60 | 44.92 (44.88 | 4.00 4.06 | 3.88 4.03) |
| 209 | Cl | Me | Br– | $Br_2$ | 64–65 | 35.15 (34.91 | 2.69 2.69 | 3.42 3.39) |
| 210 | Cl | H | Br– | $BrCH_2CH_2Br$ | 74–75 | 33.30 (33.13 | 2.28 2.28 | 3.62 3.51) |

Example 211

Preparation of 5-(4-(4-nitrophenoxy)phenylthio)-N,N-dimethyl-2-thiophenesulphonamide

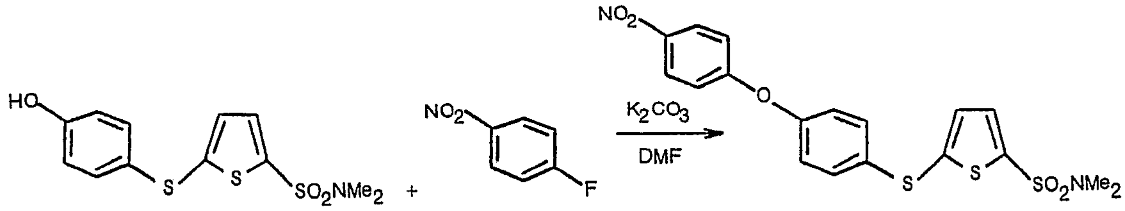


A mixture of 5-(4-hydroxyphenylthio)-N,N-dimethyl-2-thiophenesulphonamide (4 g, 0.0127 m), 4-nitrofluorobenzene (1.833 g, 0.013 m), anhydrous potassium carbonate (2.07g, 0.015 m) and anhydrous dimethylformamide (20 ml) were heated at 70° for 8 hours under an atmosphere of dry nitrogen. The reaction mixture was poured into water (200 ml) which was extracted with dichloromethane (50 ml, ×3). The combined organic layers were washed with water (100 ml ×3), sodium hydroxide solution (100 ml of 5% ×2), water (100 ml) and brine (100 ml). After drying ($MgSO_4$) the solvent was evaporated leaving a pale yellow oil which crystallized on trituration with petroleum ether giving the title compound, 3.1 g, 56%, m.p. 77—78°C.

*Analysis %:*—

| | | | |
|---|---|---|---|
| Found: | C, 49.46; | H, 3.63; | N, 6.08 |
| Required: | C, 49.52; | H, 3.69; | N, 6.42. |

Examples 212—216

The following compounds were prepared by procedures similar to that of Example 211 starting from 5-(4-hydroxyphenylthio)-N,N-dimethyl-2-thiophenesulphonamide and the stated R″.Hal:—

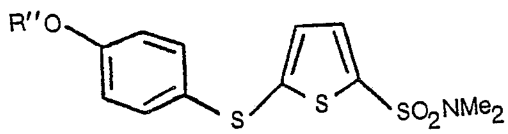

| Example No. | R″—Hal | | m.p. (°C) | Analysis % (Theoretical in brackets) C | H | N |
|---|---|---|---|---|---|---|
| 212 | 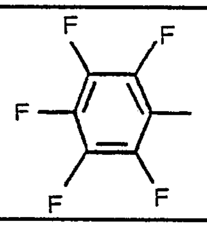 | —F | 67—68 | 44.87 (44.90 | 2.74 2.51 | 2.88 2.91) |
| 213 | $CH_3(CH_2)_3-$ | —Br | 41—41.5 | 51.50 (51.72) | 5.53 5.70 | 4.09 3.77) |
| 214 | $(CH_3)_2CH-$ | —Br | Oil | 50.04 (50.39 | 5.29 5.36 | 4.19 3.92) |
| 215 | 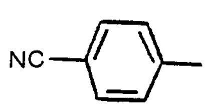 | —F | 95—96 | 54.22 (54.78 | 3.82 3.87 | 6.55 6.73) |
| 216 | 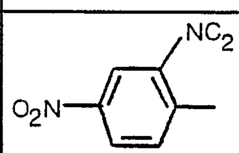 | —F | 89—93 | 44.96 (44.90 | 3.13 3.14 | 8.96 8.73) |

Example 217

Preparation of 5-(4-[N-methylcarbamoyloxy]phenylthio)-N,N-dimethyl-2-thiophenesulphonamide

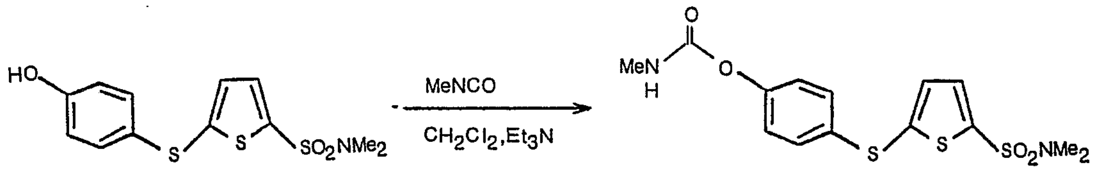


A solution of 5-(4-hydroxyphenylthio)-N,N-dimethyl-2-thiophenesulphonamide (1 g, 0.003 m), methyl isocyanate (0.4 g, 006 m), triethylamine (0.1 ml) and dry dichloromethane (10 mls) were heated under reflux for 1 hour. The solvent was then evaporated and the residue recrystallized from 60—80° petroleum ether/ethyl acetate to give the title compound 800 mg, 63%, m.p. 119—121°.

*Analysis %:—*

| | | | |
|---|---|---|---|
| Found: | 45.14; | 4.36; | 7.68. |
| Required: | 45.14; | 4.33; | 7.52. |

Example 218

Preparation of 5-(4-(4-Bromophenylthio)phenylsulphonyl)-N,N-dimethyl-2-thiophenesulphonamide

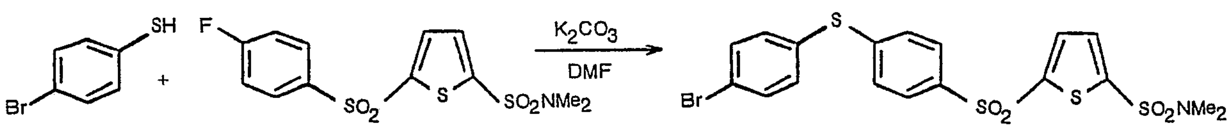


A mixture of 4-bromothiophenol (0.2 g, 0.00105 m), 5-(4-fluorophenylsulphonyl)-N,N-dimethyl-2-thiophenesulphonamide (0.35 g, 0.001 m), anhydrous potassium carbonate (0.2 g) and anhydrous dimethylformamide (5 ml) were stirred at 18° under an atmosphere of dry nitrogen for 48 hours. The mixture was poured into water (20 ml) causing a gum to precipitate. After decantation of the supernatant liquid the gum was taken up in petroleum-ether and the solution dried ($MgSO_4$). After concentration to approximately 5 ml the solution was set aside to crystallize at 0°C. The title compound was isolated by filtration, 0.2 g, 38%, m.p. 145°.

*Analysis %:—*

| | | | |
|---|---|---|---|
| Found: | C, 41.92; | H, 3.10; | N, 2.68. |
| Required: | C, 41.69; | H, 3.11; | N, 2.70. |

Example 219

The following compound was prepared in an analogous manner to that described in Example 218, using 4-bromophenol in place of 4-bromothiophenol:—

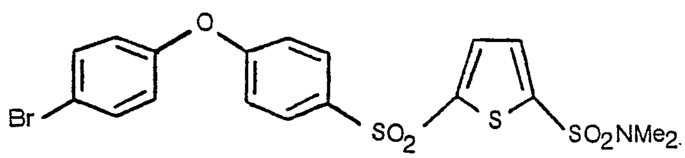

m.p. — (oily solid)

mass spectrum: $M^+$ 503 and 501 [due to Br isotopes]. $C_{18}H_{16}BrNO_5S_3$ requires M = 502.43.

Example 220

Preparation of 5-(4-dimethylaminophenylsulphonyl)-N,N-dimethyl-2-thiophenesulphonamide

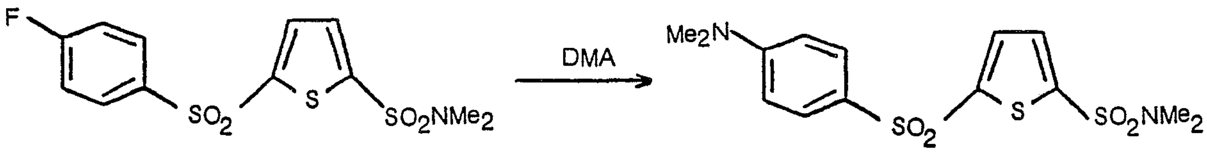


A solution of 5-(4-fluorophenylsulphonyl)-N,N-dimethyl-2-thiophenesulphonamide (0.5 g, 0.0014 m) in dry dimethylacetamide (10 ml) was heated under reflux for 48 hours and then poured into water (50 ml). The resulting precipitate was filtered off, dried and recrystallized from ethyl acetate to give the title compound 0.393 g, 74%, m.p. 175—178°.

*Analysis %:—*

| | | | |
|---|---|---|---|
| Found: | C, 44.70; | H, 4.92; | N, 7.45. |
| Required: | C, 44.90; | H, 4.84; | N, 7.48. |

Example 221

Preparaiton of 5-(4-thiocarbamoylphenylthio)-N,N-dimethyl-2-thiophenesulphonamide

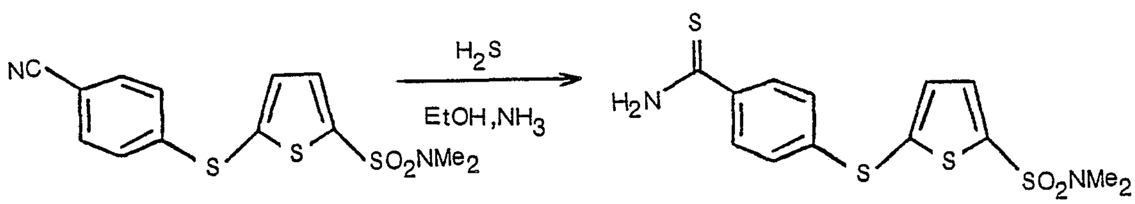


Hydrogen sulphide and ammonia gases were continuously bubbled through a solution of 5-(4-cyanophenylthio)-N,N-dimethyl-2-thiophenesulphonamide (5 g, 0.015 m) in ethanol (200 ml) for 4 hours. The solution was then allowed to stand overnight. The solution was evaporated and the solid obtained was recrystallized from toluene/ethyl acetate to give the title compound, 4 g, 73%, m.p. 150—151°.

*Analysis %:—*

| | | | |
|---|---|---|---|
| Found: | C, 43.70; | H, 3.80; | N, 8.37. |
| Required: | C, 43.55; | H, 3.94; | N, 7.81. |

Example 222

Preparation of 5-(4-(4-methyl-2-thiazolyl)phenylthio)-N,N-dimethyl-2-thiophenesulphonamide

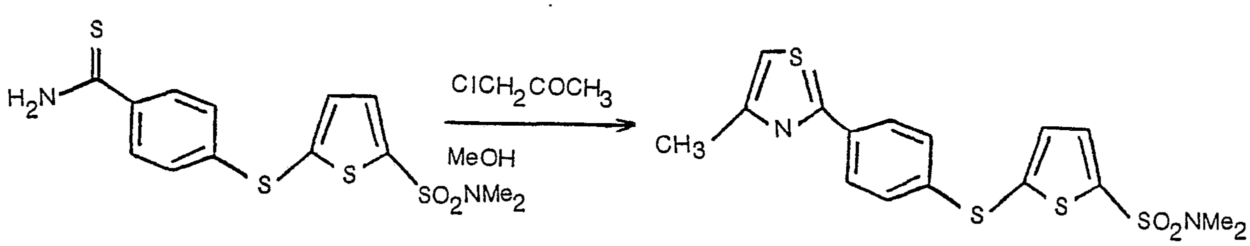


A mixture of 5-(4-thiocarbamoylphenylthio)-N,N-dimethyl-2-thiophenesulphonamide (0.3 g 0.00084 m), chloroacetone (0.1 ml 0.00125 m) and methanol (5 ml) were heated under reflux for 3 hours. The solution was cooled, the precipitate was removed by filtration and then recrystallized for 60—80° petroleum-ether/ethyl acetate to give the title compound, 0.21 g, 63%, m.p. 106°.

*Analysis %:—*

| | | | |
|---|---|---|---|
| Found: | C, 48.30; | H, 4.04; | N, 7.04. |
| Required: | C, 48.46; | H, 4.07; | N, 7.06. |

Example 223

Preparation of 5-(4-isothiocyanatophenylthio)-N,N-dimethyl-2-thiophenesulphonamide

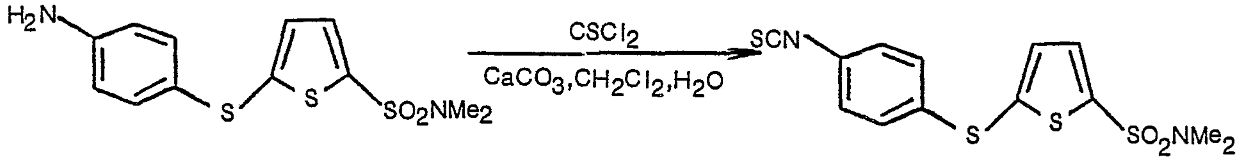


Hydrogen chloride gas was bubbled through dichloromethane (15 ml) until a saturated solution was obtained. 5-(4-Aminophenylthio)-N,N-dimethyl-2-thiophenesulphonamide (0.5 g, 0.0016 m) was added and the solution cooled to —5°. Calcium carbonate (0.25 g) and water (2 ml) were added and the mixture was stirred whilst thiophosgene (0.2 ml, 0.0026 m) in dichloromethane (10 ml) was added dropwise over a period of 30 minutes. The reaction mixture was diluted with water (20 ml) and the dichloromethane layer separated, dried ($MgSO_4$) and evaporated. The solid obtained was recrystallized from 60—80° petroleum-ether/ethyl acetate to yield the title compound, 0.425 g, 75%, m.p. 80—82°.

*Analysis %:—*

| | | | |
|---|---|---|---|
| Found: | C, 44.18; | N, 3.42; | 7.67. |
| Required: | C, 43.79; | N, 3.39; | 7.86. |

Example 224

Preparation of 5-(4-bromophenylthio)-N-thiocyanatoacetyl-N-methyl-2-thiophenesulphonamide

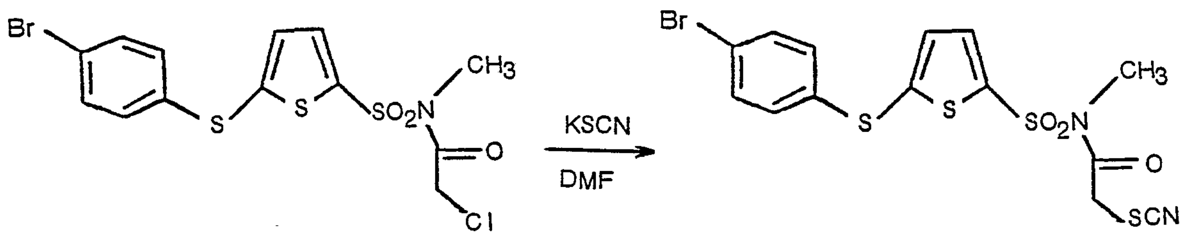


A mixture of 5-(4-bromophenylthio)-N-chloroacetyl-N-methyl-2-thiophenesulphonamide (0.3 g, 0.00068 m), potassium thiocyanate (0.3 g) and anhydrous dimethylformamide (10 ml) was stirred at room temperature for 24 hours. The mixture was poured into water and the resulting precipitate was filtered off, dried and recrystallized from 60—80° petroleum-ether/ethyl acetate to give the title compound 0.28 g, 89%, m.p. 94—5°.

*Analysis %:—*

| | | | |
|---|---|---|---|
| Found: | C, 36.26; | H, 2.36; | N, 5.93. |
| Required: | C, 36.28; | H, 2.39; | N, 6.05. |

Example 225

Preparation of 5-methoxy-N,N-dimethyl-2-thiophenesulphonamide

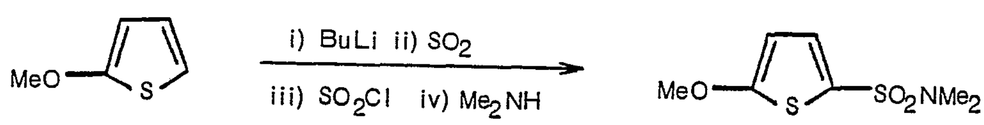


The procedure of Preparation 32 hereinafter was employed to give the title compound as an oil.

*n.m.r.* (δ) 7.23 (d, 5Hz, 1H), 6.25 (d, J 5Hz, 1H), 3.96 (s, 3H), 2.76 (s, 6H).

Example 226

Preparation of 3,5-di-(4-Bromophenylthio)-N,N-dimethyl-2-thiophenesulphonamide

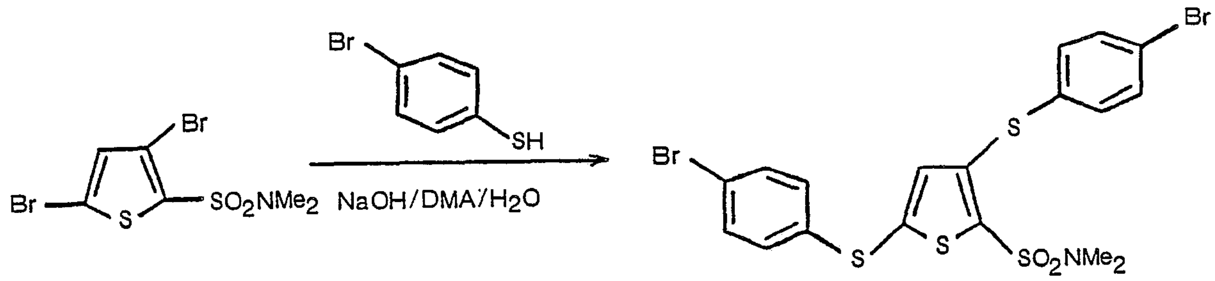


A solution of 3,5-dibromo-N,N-dimethyl-2-thiophenesulphonamide (2 g, 0.0057 m), 4-bromothiophenol (2.4 g, 0.0126 m), sodium hydroxide (0.5 g, 0.013 m), water (4 ml) and dimethylacetamide (10 ml) was heated at 100° for 4 days under an atmosphere of nitrogen. The cooled reaction mixture was poured into water (50 ml) and extracted with ethyl acetate (25 ml ×3). The combined organic layers were washed with sodium hydroxide solution (25 ml, 10%, ×2), water (25 ml ×2) brine (25 ml) and dried ($MgSO_4$). The solvent was evaporated leaving a gum which solidified on standing at 0°. Recrystallization from ethylacetate/60—80° petroleum-ether gave the title compound, 1.2 g, 36%, m.p. 127—128°.

*Analysis %:—*

| | | | |
|---|---|---|---|
| Found: | C, 38.58; | H, 2.70; | N, 2.38. |
| Required: | C, 38.23; | H, 2.67; | N, 2.48. |

Example 227

Preparation of 5-(4-Chlorophenylthio)-N-hydroxymethyl-N-methyl-2-thiophenesulphonamide

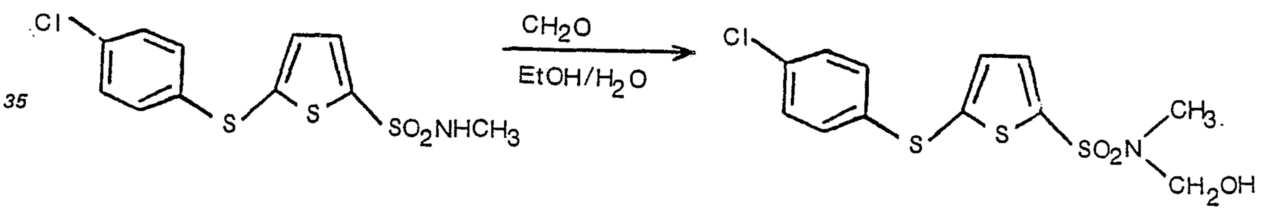


A mixture of 5-(4-chlorophenylthio)-N-methyl-2-thiophenesulphonamide (1.6 g, 0.005 m), formalin (10 ml) and ethanol (10 ml) was heated in a steam bath for 7 days. The reaction mixture was poured into water (100 ml) and the precipitated solid filtered off, dried, and recrystallized from 60—80° petroleum-ether/ethyl acetate to give the title compound 0.9 g, 52%, m.p. 73.5—77°.

*Analysis %:—*

| | | | |
|---|---|---|---|
| Found: | C, 41.02; | H, 3.39; | N, 4.11. |
| Required: | C, 41.19; | H, 3.46; | N, 4.00. |

Examples 228—230

The following compounds were prepared by procedures similar to that of Example 227, starting from the appropriate sulphonamide and formalin.

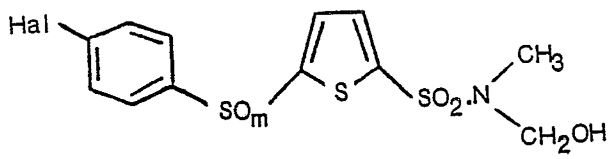

| Example No. | Hal | m | M.P. (°C) | Analysis % (Theoretical in brackets) C | H | N |
|---|---|---|---|---|---|---|
| 228 | Cl | 1 | 144—145 | 39.53 (39.39 | 2.92 3.31 | 4.23 3.83) |
| 229 | Cl | 2 | 106—110 | 37.61 (37.74 | 3.15 3.17 | 3.75 3.67) |
| 230 | Br | 0 | 84—89 | 36.55 (36.71 | 3.07 3.15 | 3.55 3.78) |

The following Preparations relate to the preparation of certain starting materials. All temperature are in °C:—

Preparation 1

Preparation of 5-Bromo-2-thiophenesulphonyl chloride

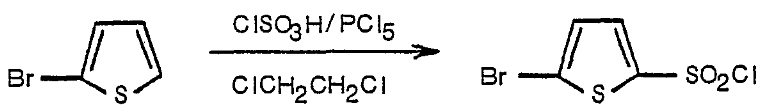


To a stirred solution of chlorsulphonic acid (57 g, 32.5 ml, 0.49 m) in 1,2-dichloroethane (130 ml) at −5° was added phosphorus pentachloride (83.2 g, 0.4 m) in small portions over a period of 30 minutes during which time the internal temperature was maintained at −5°. The mixture was stirred for a further 30 minutes. 2-Bromothiophene (65.2 g, 39 ml, 0.4 m) was added dropwise over a period of 60 minutes whilst the internal temperature was maintained at 0°. During this time hydrogen chloride gas was evolved. The mixture was stirred at 0° for a further 2 hours and then warmed at 80°C for 1 hour. The resulting clear purple solution was cooled to room temperature, poured onto ice (1 kg) and the mixture allowed to stand for 1 hour. The aqueous suspension was extracted with chloroform (500 ml, ×3). The combined organic extracts were dried over anhydrous magnesium sulphate, filtered and evaporated *in vacuo* to give 5-bromo-2-thiophenesulphonyl chloride as a yellow-brown oil (100 g, 96%) which was used without further purification.

*n.m.r.*

$\delta(CDCl_3)$: 7.15 (d, J 5Hz, 1H), 7.65 (d, J 5Hz, 1H)

Preparations 2 and 3

The following compounds were prepared by procedures similar to that of Preparation 1, starting from the appropriate bromothiophene and chlorsulphonic acid/$PCl_5$:—

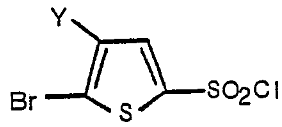

| Preparation No. | Y | n.m.r. (δ) |
|---|---|---|
| 2 | Br | 7.7 (s,1H) |
| 3 | $CH_3$ | Not characterised, used without further purification |

Preparation 4

Preparation of 5-Bromo-N-methyl-2-thiophenesulphonamide

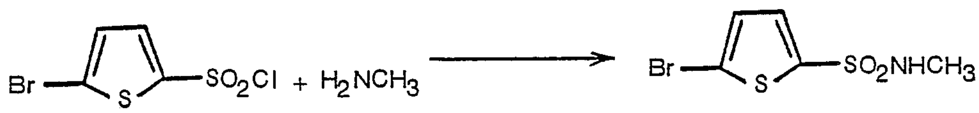

To a stirred solution of 5-bromo-2-thiophenesulphonyl chloride (100 g, 0.382 m) in absolute alcohol (250 ml) at —40° was added an alcoholic solution of methylamine (75 ml, 33% w/v, 0.8 m) dropwise over a period of 1 hour. The reaction mixture was allowed to warm to room temperature and then left overnight during which time a precipitate formed. The precipitate was removed by filtration and the filtrate was evaporated *in vacuo* to leave an oil. The oil was taken up in ether (750 ml) and dilute hydrochloric acid (200 ml, 10%). The ether layer was separated, washed with saturated sodium hydrogen, carbonate solution (200 ml), saturated sodium chloride solution (200 ml), dried over anhydrous magnesium sulphate and evaporated *in vacuo* to give an oil. The oil was taken up in ether (200 ml) and cooled in an ice-bath. Petroleum ether (300 ml, b.p. 40°—60°) was slowly added to the stirred solution and the mixture was set aside. 5-Bromo-N-methyl-2-thiophenesulphonamide crystallised out and was isolated by filtration (83 g, 85%), m.p. 58—60°C.

*Analysis %:*—

| | | | |
|---|---|---|---|
| Found: | C, 23.22; | H, 2.24; | N, 5.54. |
| Required: | C, 23.45; | H, 2.34; | N, 5.47 |

Preparations 5—31

The following compounds were prepared by procedures similar to that of Preparation 4, starting from the appropriate thiophenesulphonyl chloride and amine:—

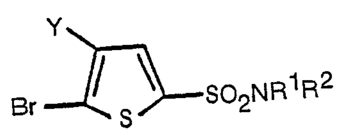

| Preparation No. | $R^1$ | $R^2$ | Y | m.p. (°C) | Analysis % (or n.m.r.) (Theoretical in Brackets) C | H | N |
|---|---|---|---|---|---|---|---|
| 5 | H | Et | H | 59–60 | 26.96 (26.67 | 2.98 2.98 | 5.22 5.18) |
| 6 | H | *i*-Pr | H | 68–69 | 29.62 (29.58 | 3.55 3.54 | 5.05 4.93) |
| 7 | Me | Me | H | 45–48 | 26.07 (26.68 | 2.86 2.96 | 6.65 5.18) |
| 8 | Me | Et | H | Oil | 29.30 (29.58 | 3.51 3.55 | 4.75 4.93) |
| 9 | Me | *i*-Pr | H | Oil | 32.49 (32.22 | 4.18 4.06 | 4.85 4.70) |
| 10 | Me | *t*-Bu | H | 61–62 | 34.39 (34.62 | 4.43 4.52 | 4.82 4.49) |
| 11 | Me | $-CH_2CH{=}CH_2$ | H | 26–28 | 32.21 (32.04 | 3.29 3.40 | 4.79 4.73) |
| 12 | Me | cyclopentyl | H | Oil | 36.95 (37.04 | 4.37 4.35 | 4.14 4.32) |
| 13 | Me | cyclohexyl | H | Oil | 39.27 (39.17 | 4.74 4.48 | 4.32 4.15) |
| 14 | Me | phenyl | H | 84 | 39.89 (29.76 | 2.87 3.03 | 4.52 4.21) |
| 15 | Me | benzyl | H | 53–55 | 41.58 (41.62 | 3.43 3.49 | 4.06 4.04) |
| 16 | Et | Et | H | Oil | 31.85 (32.22 | 3.96 4.01 | 5.06 4.70) |
| 17 | 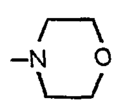 | | H | 109–110 | 30.92 (30.77 | 3.15 3.23 | 4.51 4.49) |
| 18 | 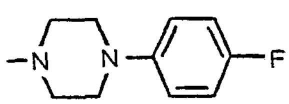 | | H | 128 | 41.47 (41.48 | 3.40 3.48 | 6.86 6.91) |
| 19 | 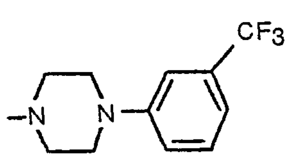 | | H | 110 | 39.46 (39.56 | 3.03 3.09 | 6.12 6.15) |

| Preparation No. | $R^1$ | $R^2$ | Y | m.p. (°C) | Analysis % (or n.m.r.) (Theoretical in Brackets) C | H | N |
|---|---|---|---|---|---|---|---|
| 20 | 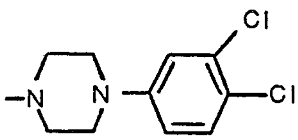 | | H | 134 | 36.80 (36.85 | 2.79 2.87 | 6.08 6.14) |
| 21 | Me | Me | Br | 113–115 | 20.60 (20.64 | 1.76 2.02 | 4.11 4.01) |
| 22 | Me | Me | $CH_3$ | 69–70.5 | 29.91 (29.58 | 3.49 3.55 | 5.21 4.93) |
| 23 | H | cyclopropyl | H | 75–76 | 30.13 (29.79 | 2.80 2.86 | 5.03 4.96) |
| 24 | 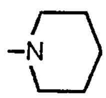 | | H | 43–44 | 34.94 (34.84 | 3.86 3.90 | 4.57 4.52) |
| 25 | 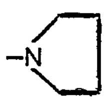 | | H | 67–68 | 32.49 (32.44 | 3.27 3.40 | 4.73 4.73) |
| 26 | Et | Et | H | b.p. 120–131 0.5 mm Hg | 31.50 (32.22 | 3.93 4.01 | 5.13 4.70) |
| 27 | Me | OMe | H | Oil | 7.35(d,ArH), 7.05(d,ArH), 3.8(s,$OCH_3$), 2.8(s,$NCH_3$) | | |
| 28 | H | 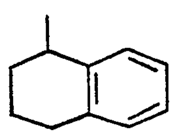 | H | 102–4 | 45.08 (45.16 | 3.75 3.79 | 3.77 3.76) |
| 29 | H | $CF_3CH_2$– | H | Oil | no analytical data | | |
| 30 | H | - cyclopropyl | H | 75–76 | 30.13 (29.79 | 2.80 2.86 | 5.03 4.96) |
| 31 | Me | 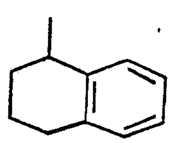 | H | 68–71 | 46.70 (46.63 | 4.14 4.17 | 3.49* 3.63) |

*This compound was also prepared by the methylation of the product of Preparation 28 with methyl iodide.

Preparation 32

Preparation of 3,5-dibromo-N,N-dimethyl-2-thiophenesulphonamide

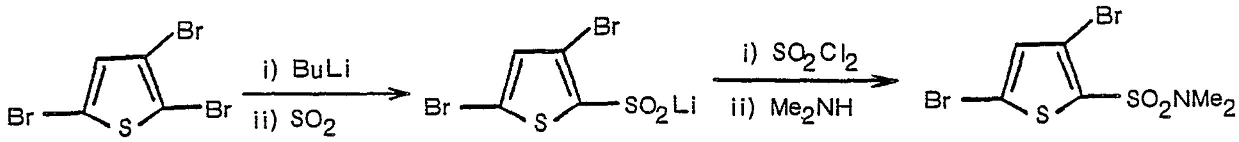


A solution of 2,3,5-tribromothiophene (12.8 g, 0.04 m) in anhydrous ether (70 ml) was cooled to −78°C under an atmosphere of dry nitrogen. n-Butyllithium (27.5 ml of 1.6 M solution in hexane, 0.044 m) was added at such a rate that the internal temperature was maintained below −60°. The resulting mixture was stirred for 30 minutes. An excess of sulphur dioxide was condensed into the reaction vessel and the mixture was allowed to warm to room temperature. The precipitated sulphinate salt was isolated by filtration, suspended in dichloromethane (100 ml) which was then cooled to −78°. Sulphuryl chloride (7.5 ml, 0.093 m) was added dropwise to the stirred solution at such a rate that the internal temperature was maintained below −45°. An excess of dimethylamine was then added until the resulting solution was basic. The dichloromethane was extracted with water and then dried ($MgSO_4$). Evaporation of the solvent gave a crude oil which was purified by chromatography on "Whatman" (Trade Mark) S13 TLC grade silica eluted with dichloromethane. Combination of the relevant fractions gave, after evaporation, the title compound, 5.7 g, 41% as an oil.

*n.m.r.* ($\delta$): 7.1 (s, H, Ar-H), 2.9 (s, 6H, $NMe_2$).

Activity data ($LD_{90}$'s) for the compounds of the Examples against *Lucilia sericata* larvae, *Lucilia cuprina* larvae and *Musca domestica* larvae is as follows:—

| Example No. | $LD_{90}$ versus | | |
|---|---|---|---|
| | Lucilia sericata (Mg $m^{-2}$) | Lucilia cuprina (Mg $m^{-2}$) | Musca domestica ($\mu g\ ml^{-1}$) |
| 1* | 6.25 | – | – |
| 2 | >25 | – | 100 |
| 3 | >100 | – | >100 |
| 4 | >25 | – | >100 |
| 5 | 3.125 | – | >100 |
| 6 | – | >12.5 | 100 |
| 7 | – | >100 | >100 |
| 8 | – | >100 | >100 |
| 9 | – | >100 | >100 |
| 10 | 100 | – | – |
| 11 | >100 | – | – |
| 12 | >100 | – | – |
| 13 | 3.125 | – | – |
| 14 | 3.125 | 12.5 | – |
| 15 | 1.56 | – | – |
| 16 | 6.25 | – | 100 |
| 17 | >25 | – | >100 |
| 18 | >25 | – | – |
| 19 | 12.5 | – | – |
| 20 | >25 | – | >100 |

* $LD_{90}$ against *Boophilus microplus* larvae was 12.5 $mg.m^{-2}$.

| Example No. | $LD_{90}$ versus | | |
|---|---|---|---|
| | Lucilia sericata (Mg $m^{-2}$) | Lucilia cuprina (Mg $m^{-2}$) | Musca domestica ($\mu g$ $ml^{-1}$) |
| 21 | >100 | – | 100 |
| 22 | >25 | – | >100 |
| 23 | – | – | – |
| 24 | >100 | – | – |
| 25 | >25 | – | >100 |
| 26 | 25 | – | >100 |
| 27 | >25 | – | – |
| 28 | >25 | – | – |
| 29 | >25 | – | – |
| 30 | 25 | – | – |
| 31 | 6.25 | – | – |
| 32 | >25 | – | – |
| 33 | 12.5 | – | 100 |
| 34 | 3.125 | – | – |
| 35 | 3.125 | – | >100 |
| 36 | 6.25 | – | 12 |
| 37 | 6.25 | – | 50 |
| 38 | – | 12.5 | 25 |
| 39 | 12.5 | – | 50 |
| 40 | >12.5 | – | >100 |

| Example No. | LD$_{90}$ versus Lucilia sericata (Mg $m^{-2}$) | Lucilia cuprina (Mg $m^{-2}$) | Musca domestica ($\mu g$ $ml^{-1}$) |
|---|---|---|---|
| 41 | 12.5 | – | 100 |
| 42 | 12.5 | – | >100 |
| 43 | >12.5 | – | >100 |
| 44 | >25 | – | >100 |
| 45 | 6.25 | 12.5 | 50 |
| 46 | 6.25 | – | 100 |
| 47 | >12.5 | – | >100 |
| 48 | >25 | – | >100 |
| 49 | >25 | – | >100 |
| 50 | >25 | – | >100 |
| 51 | 12.5 | – | 25 |
| 52 | 25 | – | 25 |
| 53 | 100 | – | >100 |
| 54 | 12.5 | – | 100 |
| 55 | >12.5 | – | 25 |
| 56 | >12.5 | – | >100 |
| 57 | >12.5 | – | >100 |
| 58 | >12.5 | – | >100 |
| 59 | >12.5 | – | >100 |
| 60 | >100 | – | >100 |

| Example No. | LD$_{90}$ versus | | |
|---|---|---|---|
| | Lucilia sericata (Mg m$^{-2}$) | Lucilia cuprina (Mg m$^{-2}$) | Musca domestica ($\mu$g ml$^{-1}$) |
| 61 | 12.5 | 12.5 | 12.5 |
| 62 | – | >12.5 | >50 |
| 63 | >100 | – | >50 |
| 64 | – | – | – |
| 65 | – | >12.5 | 100 |
| 66 | – | 100 | >100 |
| 67 | – | 100 | 50 |
| 68 | – | >12.5 | >100 |
| 69 | – | >12.5 | >100 |
| 70 | 12.5 | – | >100 |
| 71a | 6.25 | – | 50 |
| 71b | 12.5 | – | 100 |
| 72 | >12.5 | – | >100 |
| 73 | 3.125 | – | 25 |
| 74 | 6.25 | – | 100 |
| 75 | 6.25 | – | 100 |
| 76 | 12.5 | – | 50 |
| 77 | 6.25 | – | 100 |
| 78 | 12.5 | – | 50 |
| 79 | 12.5 | – | >100 |
| 80 | 3.125 | – | 100 |

| Example No. | LD$_{90}$ versus Lucilia sericata (Mg m$^{-2}$) | Lucilia cuprina (Mg m$^{-2}$) | Musca domestica ($\mu$g ml$^{-1}$) |
|---|---|---|---|
| 81 | – | 6.25 | 100 |
| 82 | – | >12.5 | >100 |
| 83 | – | >12.5 | >100 |
| 84 | – | >12.5 | >100 |
| 85 | – | >12.5 | >100 |
| 86 | – | >12.5 | >100 |
| 87 | – | >12.5 | >100 |
| 88 | – | >12.5 | >100 |
| 89 | – | >12.5 | >100 |
| 90 | – | >12.5 | >100 |
| 91 | – | >12.5 | >100 |
| 92 | – | >12.5 | >100 |
| 93 | <12.5 | – | >50 |
| 94 | – | >12.5 | >100 |
| 95 | >12.5 | – | >50 |
| 96 | >12.5 | – | >50 |
| 97 | – | 12.5 | 100 |
| 98 | – | 12.5 | 100 |
| 99 | – | 12.5 | 100 |
| 100 | – | >12.5 | >100 |

| Example No. | $LD_{90}$ versus | | |
|---|---|---|---|
| | Lucilia sericata (Mg $m^{-2}$) | Lucilia cuprina (Mg $m^{-2}$) | Musca domestica ($\mu g\ ml^{-1}$) |
| 101 | – | 12.5 | 100 |
| 102 | – | 12.5 | 100 |
| 103 | – | 12.5 | 100 |
| 104 | – | >12.5 | >100 |
| 105 | – | 12.5 | 100 |
| 106 | – | 12.5 | >100 |
| 107 | – | >12.5 | 100 |
| 108 | – | 12.5 | 100 |
| 109 | – | 100 | 50 |
| 110 | – | >12.5 | 100 |
| 111 | – | >12.5 | >100 |
| 112 | – | 12.5 | 100 |
| 113 | – | 12.5 | 100 |
| 114 | – | >12.5 | 100 |
| 115 | >12.5 | – | 12.5 |
| 116 | 12.5 | – | 100 |
| 117 | – | 6.25 | 50 |
| 118 | – | 100 | >100 |
| 119 | >25 | – | >100 |
| 120 | >100 | – | 100 |

| Example No. | $LD_{90}$ versus | | |
|---|---|---|---|
| | Lucilia sericata (Mg $m^{-2}$) | Lucilia cuprina (Mg $m^{-2}$) | Musca domestica ($\mu$g $ml^{-1}$) |
| 121 | 500 | – | >50 |
| 122 | >25 | – | 100 |
| 123 | >500 | – | >50 |
| 124 | – | – | – |
| 125 | – | 6.25 | 50 |
| 126 | – | >12.5 | – |
| 127 | – | >12.5 | >100 |
| 128 | – | >12.5 | – |
| 129 | 6.25 | – | – |
| 130 | 3.125 | – | – |
| 131 | >100 | – | – |
| 132 | >100 | – | – |
| 133 | >25 | – | – |
| 134 | 12:5 | – | 25 |
| 135 | – | 12.5 | 100 |
| 136 | 25 | – | – |
| 137 | 100 | – | – |
| 138 | >100 | – | – |
| 139 | >100 | – | – |
| 140 | 25 | 6.25 | – |

| Example No. | LD$_{90}$ versus | | |
|---|---|---|---|
| | Lucilia sericata (Mg m$^{-2}$) | Lucilia cuprina (Mg m$^{-2}$) | Musca. domestica ($\mu$g ml$^{-1}$) |
| 141 | 1.56 | 6.25 | >50 |
| 142 | 25 | – | 100 |
| 143 | >25 | – | >100 |
| 144 | >100 | – | >100 |
| 145 | >25 | – | >100 |
| 146 | 12.5 | – | >100 |
| 147 | >25 | – | >100 |
| 148 | >100 | – | – |
| 149 | >25 | – | >100 |
| 150 | 25 | – | – |
| 151 | >25 | – | – |
| 152 | >25 | – | – |
| 153 | 25 | – | – |
| 154 | >25 | – | – |
| 155 | 12.5 | – | 100 |
| 156 | 12.5 | – | 50 |
| 157 | 6.25 | – | 100 |
| 158 | 6.25 | – | 100 |
| 159 | 6.25 | – | 100 |
| 160 | 6.25 | – | 50 |

| Example No. | LD$_{90}$ versus Lucilia sericata (Mg $m^{-2}$) | Lucilia cuprina (mg $m^{-2}$) | Musca domestica ($\mu$g $ml^{-1}$) |
|---|---|---|---|
| 161 | >12.5 | – | >100 |
| 162 | 12.5 | – | 100 |
| 163 | 25 | – | 100 |
| 164 | >12.5 | – | >100 |
| 165 | >12.5 | – | >100 |
| 166 | >25 | – | >100 |
| 167 | 25 | – | >100 |
| 168 | 12.5 | – | 25 |
| 169 | >25 | – | >100 |
| 170 | >25 | – | >100 |
| 171 | >25 | – | >100 |
| 172 | 12.5 | – | – |
| 173 | 6.25 | – | >100 |
| 174 | >12.5 | – | 100 |
| 175 | 3.125 | – | 100 |
| 176 | >12.5 | – | 100 |
| 177 | >12.5 | – | >100 |
| 178 | >12.5 | – | >100 |
| 179 | >12.5 | – | >100 |
| 180 | – | 6.25 | 100 |

| Example No. | LD$_{90}$ versus | | |
|---|---|---|---|
| | Lucilia sericata (Mg $m^{-2}$) | Lucilia cuprina (Mg $m^{-2}$) | Musca domestica ($\mu g$ $ml^{-1}$) |
| 181 | – | 6.25 | >100 |
| 182 | – | >12.5 | 50 |
| 183 | – | >12.5 | 100 |
| 184 | – | – | – |
| 185 | – | 12.5 | 12 |
| 186 | 500 | – | >100 |
| 187 | 25 | – | >100 |
| 188 | >12.5 | – | >100 |
| 189 | 25 | – | 50 |
| 190 | 25 | – | 100 |
| 191 | >25 | – | >100 |
| 192 | >25 | – | >100 |
| 193 | 25 | – | 25 |
| 194 | 25 | – | >100 |
| 195 | >25 | – | 100 |
| 196 | >25 | – | >100 |
| 197 | – | – | – |
| 198 | 12.5 | – | 100 |
| 199 | >12.5 | – | >100 |
| 200 | >12.5 | – | >100 |

| Example No. | LD$_{90}$ versus | | |
|---|---|---|---|
| | Lucilia sericata (Mg $m^{-2}$) | Lucilia cuprina (Mg $m^{-2}$) | Musca domestica ($\mu g\ ml^{-1}$) |
| 201 | >100 | – | >100 |
| 202 | >12.5 | – | >100 |
| 203 | >12.5 | – | >100 |
| 204 | – | >12.5 | >100 |
| 205 | – | >12.5 | – |
| 206 | – | >12.5 | – |
| 207 | – | >12.5 | >100 |
| 208 | – | >12.5 | >100 |
| 209 | – | >12.5 | >100 |
| 210 | >12.5 | – | 25 |
| 211 | >12.5 | – | >50 |
| 212 | – | >12.5 | >100 |
| 213 | – | >12.5 | >100 |
| 214 | – | >12.5 | 50 |
| 215 | – | >12.5 | >100 |
| 216 | – | >12.5 | >100 |
| 217 | – | >12.5 | >100 |
| 218 | – | >12.5 | >100 |
| 219 | >12.5 | – | >100 |
| 220 | – | >12.5 | >100 |

| Example No. | LD$_{90}$ versus | | |
|---|---|---|---|
| | Lucilia sericata (Mg $m^{-2}$) | Lucilia cuprina (Mg $m^{-2}$) | Musca domestica ($\mu g$ $ml^{-1}$) |
| 221 | – | >12.5 | >100 |
| 222 | – | >12.5 | >100 |
| 223 | – | 12.5 | 100 |
| 224 | – | >100 | >100 |
| 225 | – | >12.5 | >100 |
| 226 | – | 12.5 | 25 |
| 227 | – | 6.25 | 100 |
| 228 | – | 6.25 | 100 |
| 229 | – | >100 | >100 |
| 230 | – | >100 | >100 |

**Claims**

1. Compounds of the formula:

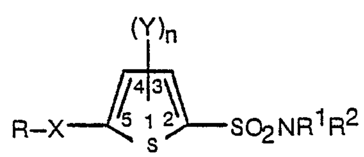

—— (I)

and their alkali metal or non-toxic acid addition salts, wherein:

(a) $R_1$ is hydrogen or lower alkyl;

and $R^2$ is lower alkyl, lower alkoxy, 2,2,2-trifluoroethyl, hydroxy-lower alkyl, 2-methoxyethoxymethyl, lower alkoxycarbonyl-lower alkyl, lower alkanoyloxy-lower alkyl, aryl-lower alkyl, aryloxy-lower alkyl, thiocyanatoacetyl, lower cycloalkyl-lower alkyl, lower cycloalkyl, lower cycloalkenyl, lower alkenyl, allenyl, lower alkynyl, $C_2$—$C_{16}$ alkanoyl, halo-lower alkanoyl, aryl-lower alkanoyl, N,N-di(lower alkyl)sulfamoyl, 1,2,3,4-tetrahydronaphthyl, arylthio, aryl or aroyl;

or $R^1$ and $R^2$ together with the nitrogen atom to which they are attached form a saturated 5- or 6-membered heterocyclic group optionally containing a further heteroatom selected from O, S and N, said N atom bearing a hydrogen atom or a lower alkyl or aryl group, said heterocyclic group optionally being substituted on a carbon atom by 1 or 2 lower alkyl groups;

(b) X is O, S, SO or $SO_2$;

(c) Y is halogen, lower alkyl, tri(lower alkyl)silyl, lower alkoxy, or a group of the formula $R^3S$— or $R^3SO_2$— wherein $R^3$ is lower alkyl or aryl; n is 0 or 1; *and*

(d) R is a lower alkyl, lower alkenyl, lower cycloalkyl, allenyl, lower alkoxy-lower alkyl, aryl-lower alkyl, aryl, heteroaromatic or heteroaromatic-phenyl group, with the proviso that when X is $SO_2$, R can also be —$NR^1R^2$ wherein $R^1$ and $R^2$ are as defined above.

the term "lower" as applied to an alkyl, alkoxy, alkanoyl, alkenyl or alkynyl group means that said group has up to 6 carbon atoms; "lower" as applied to a cycloalkyl group means that said group has from 3 to 7 carbon atoms; "lower" as applied to a cycloalkenyl group means that said group has from 4 to 7 carbon atoms;

and the term "aryl" means unsubstituted phenyl, unsubstituted naphthyl or substituted phenyl.

2. A compound as claimed in claim 1 wherein substituted phenyl is either (a) phenyl substituted by 1 or 2 substituents selected from lower alkyl, lower alkoxy, hydroxy, halogen, trifluoromethyl, cyano, amino, di(lower alkyl) amino, nitro, lower alkylthio, lower alkylsulphinyl, lower alkylsulphonyl, thiocarbamoyl, isothiocyanato, methylenedioxy, sulphamoyl, N,N-di(lower alkyl) sulphamoyl, and N-(lower alkyl)carbamoyloxy (b) pentafluorophenyl (c) biphenylyl or (d) phenyl monosubstituted by phenoxy, cyanophenoxy, halophenoxy, nitrophenoxy, dinitrophenoxy, pentafluorophenoxy or halophenylthio.

3. A compound as claimed in claim 1 or 2 wherein by heteroaromatic is meant thienyl, pyridyl, imidazolyl, benzthiazolyl, benzoxazolyl, pyrimidinyl, quinolyl, thiazolyl or pyridazinyl, optionally containing one or two substituents selected from lower alkyl, lower alkoxy and halogen.

4. A compound as claimed in claim 1, 2 or 3 wherein when $R^1$ and $R^2$ together with the nitrogen atom to which they are attached form a heterocyclic group as defined in claim 1, this group is a 1-pyrrolidinyl, piperidino, morpholino or piperazino group, said morpholino group optionally being substituted by one or two methyl groups, and said piperazino group optionally being substituted in the 4-position by a phenyl group which may itself be substituted by 1 or 2 halogen or $CF_3$ groups.

5. A compound as claimed in claim 1 wherein

(a) $R^1$ is hydrogen or $C_1$—$C_4$ alkyl;

and $R^2$ is $C_1$—$C_4$ alkyl, methoxy, 2,2,2-trifluoroethyl, hydroxymethyl, 2-methoxyethoxymethyl, $C_1$—$C_4$ alkoxycarbonylmethyl, $C_2$—$C_5$ alkanoyloxymethyl, benzyl; benzyl mono- or di-substituted on the phenyl ring portion by $C_1$—$C_4$ alkyl or halogen or mono-substituted by methylenedioxy; pentafluorophenylmethyl, phenethyl, phenoxy-($C_1$—$C_4$ alkyl), thiocyanatoacetyl, cyclopropylmethyl, $C_3$—$C_6$ cycloalkyl, cyclohexenyl, allyl, allenyl, propargyl, $C_1$—$C_{16}$ alkanoyl, haloacetyl, phenylacetyl, N,N-dimethylsulphamoyl, 1,2,3,4-tetrahydronaphth-1-yl, phenylthio, benzoyl optionally mono- or di-substituted by $C_1$—$C_4$ alkyl, $C_1$—$C_4$ alkoxy or halogen; or phenyl;

or $R^1$ and $R^2$ taken together with the nitrogen atom to which they are attached represent a 1-pyrrolidinyl, piperidino, morpholino or piperazino group, said morpholino group optionally being substituted by 1 or 2 methyl groups, and said piperazino group optionally being substituted in the 4-position by a phenyl group which may itself be substituted by 1 or 2 halogen or $CF_3$ groups;

(b) X is O, S, SO or $SO_2$;

(c) Y is halogen, $C_1$—$C_4$ alkyl, trimethylsilyl, $C_1$—$C_4$ alkoxy or a group of the formula $R^3S$— where $R^3$ is $C_1$—$C_4$ alkyl or halophenyl; n is 0 or 1; *and*

(d) R is $C_1$—$C_4$ alkyl, allyl, $C_3$—$C_6$ cycloalkyl, allenyl, methoxymethyl, phenethyl, naphthyl, biphenyl, thienyl, pyridyl, halopyridyl, dihaloimidazolyl, quinolyl, benzoxazolyl, benzthiazolyl, methoxypyridazinyl; phenyl; phenyl mono- or di-substituted by $C_1$—$C_4$ alkyl, $C_1$—$C_4$ alkoxy, halogen, cyano, trifluoromethyl, hydroxy, sulphamoyl, N,N-dimethylsulfamoyl, amino, dimethylamino, nitro, methylthio, methylsulphinyl, methylsulphonyl, N-methylcarbamoyloxy, isothiocyanato or thiocarbamoyl; or phenyl monosubstituted by phenoxy, pentafluorophenoxy, cyanophenoxy, nitrophenoxy, dinitrophenoxy, halophenoxy, halophenylthio or 4-methyl-2-thiazalyl; with the proviso that when X is $SO_2$, R can also be —$NR^1R^2$ wherein $R^1$ is $C_1$—$C_4$ alkyl, and $R^2$ is $C_1$—$C_4$ alkyl, cyclopropyl or allyl, or $R^1$ and $R^2$ taken together with the nitrogen atom to which they are attached represent a morpholino group optionally substituted by 1 or 2 methyl groups.

6. A compound as claimed in claim 1 of the formula:—

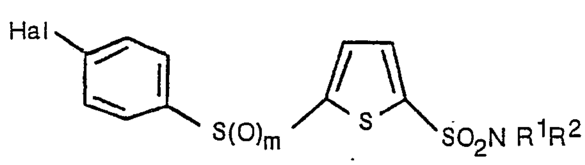

--- (IA)

wherein $R^1$ is hydrogen or $C_1$—$C_4$ alkyl, $R^2$ is $C_1$—$C_4$ alkyl, $C_3$—$C_6$ cycloalkyl, $C_2$—$C_4$ alkanoyl or benzoyl, "Hal" is Cl or Br, and m is 0 or 2.

7. A compound as claimed in claim 1 of the formula:—

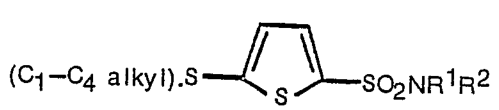

--- (IB)

wherein $R^1$ and $R^2$ are as defined in claim 6.

8. A compound as claimed in claim 1 of the formula:—

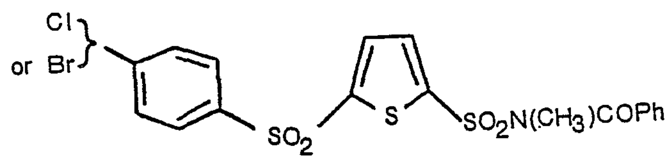

9. A compound of the formula (I) as defined in claim 1 and its alkali metal or non-toxic acid addition salts:—

wherein:—

(a) $R^1$ is hydrogen or lower alkyl;

and $R^2$ is lower alkyl, lower alkoxycarbonyl-lower alkyl, lower alkanoyloxy-lower alkyl, aryl-lower alkyl, lower cycloalkyl-lower alkyl, lower cycloalkyl, lower cycloalkenyl, lower alkenyl, allenyl, lower alkynyl, lower alkanoyl, halo-lower alkanoyl, aryl, aroyl, arylthio or lower alkoxy; or $R^1$ and $R^2$ together with the nitrogen atom to which they are attached form a saturated 5- or 6-membered heterocyclic group optionally containing a further heteroatom selected from O, S and N, said N atom bearing a hydrogen atom or a lower alkyl or aryl group, said heterocyclic group optionally being substituted on a carbon atom by 1 or 2 lower alkyl groups;

(b) X is O, S, SO or $SO_2$;

(c) Y is halogen, lower alkyl, lower alkoxy, tri(lower alkyl)silyl or a group of the formula $R^3S$— or $R^3SO_2$— wherein $R^3$ is lower alkyl or aryl; n is 0 or 1; *and*

(d) R is a lower alkyl, lower alkenyl, allenyl, lower alkoxy-lower alkyl, aryl-lower alkyl, aryl or heteroaromatic group, with the proviso that when X is $SO_2$ may also be —$NR^1R^2$ wherein $R^1$ and $R^2$ are as defined above;

"aryl" means unsubstituted phenyl, unsubstituted naphthyl, or phenyl substituted by 1 or 2 substituents selected from lower alkyl, lower alkoxy, hydroxy, halogen, trifluoromethyl, cyano, nitro, lower alkylthio, lower alkylsulphinyl, lower alkylsulphonyl, and N,N-di(lower alkyl)sulphamoyl; and "lower" is as defined in claim 1.

10. A process for preparing a compound of the formula (I) as claimed in claim 1 in which X is S, which comprises reacting a compound of the formula:—

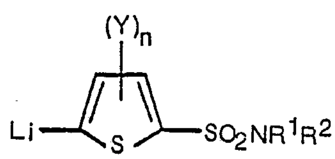

wherein Y, $R^1$, $R^2$ and n are as defined in claim 1, with a disulphide of the formula:—

RS—SR

wherein R is as defined in claim 1.

11. A process of preparing a compound of the formula (I) as claimed in claim 1 in which X is O or S and R is an aryl or heteroaromatic group, which comprises reacting, in an organic solvent and in the presence of a base, a compound of the formula:

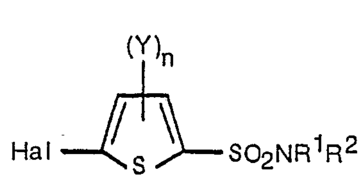

wherein Y, $R^1$, $R^2$ and n are as defined in claim 1 and "Hal" is Br, Cl or I, with a compound of the formula:

RXH

wherein R and X are as defined in this method.

12. A process for preparing a compound of the formula (I) as claimed in claim 1 wherein $R^2$ is other than aryl, and $R^1$ and $R^2$ taken together with the nitrogen atom to which they are attached do not form a ring, which comprises reacting, in the presence of a base, a compound of the formula:—

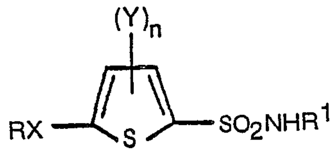

with a compound of the formula $R^2$.Hal
wherein R, X, Y and n are as defined in claim 1, $R^1$ and $R^2$ are as defined in this method, and "Hal" is Br, Cl or I.

13. A process for preparing a compound of the formula (I) as claimed in claim 1 in which $R^1$ is lower alkyl, which comprises reacting the corresponding compound of the formula (I) in which $R^1$ is H with a lower alkyl halide in the presence of a base.

14. A process for preparing a compound of the formula (I) as claimed in claim 1 wherein $R^1$ and $R^2$ are both lower alkyl, which comprises reacting the corresponding compound of the formula (I) in which $R^1$ and $R^2$ are both hydrogen with at least 2 equivalents of a lower alkyl halide in the presence of a base.

15. A process for preparing a compound of the formula (I) as claimed in claim 1 in which X is S, which comprises reacting a compound of the formula:—

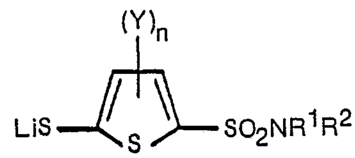

with a compound of the formula R.Hal
where Hal is Br, Cl or I and R, $R^1$, $R^2$, Y and n are as defined in claim 1.

16. A process for preparing a compound of the formula (I) as claimed in claim 1 in which X is SO or $SO_2$, which comprises oxidising the corresponding compound in which X is S using, respectively, *m*-chloroperbenzoic acid, or hydrogen peroxide in glacial acetic acid.

17. A process for preparing a compound of the formula (I) as claimed in claim 1, which comprises reacting a compound of the formula:—

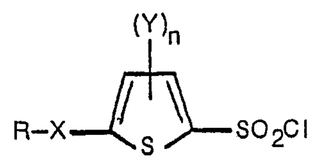

with an amine of the formula:— $R^1R^2NH$,
R, $R^1$, $R^2$, X, Y and n being as defined in claim 1.

18. An insecticidal or larvicidal composition or concentrate comprising a compound of the formula (I) or alkali metal or non-toxic acid addition salt thereof as claimed in claim 1 together with a non-toxic diluent or carrier.

**Patentansprüche**

1. Verbindungen der Formel

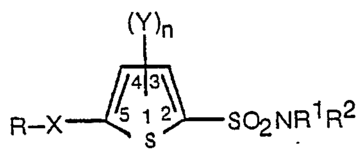 --- (I)

und deren Alkali- oder nicht-toxischen Säureadditionssalze, in welcher:
(a) $R^1$ Wasserstoff oder Nieder-Alkyl bedeutet;
und $R^2$ Nieder-Alkyl, Nieder-Alkoxy, 2,2,2-Trifluoroethyl, Hydroxy-niederalkyl, 2-Methoxyethoxymethyl, Nieder-Alkoxycarbonyl-niederalkyl, Nieder-Alkanoyloxy-niederalkyl, Aryl-niederalkyl, Aryloxyniederalkyl, Thiocyanatoacetyl, Nieder-Cycloalkyl-niederalkyl, Nieder-Cycloalkyl, Nieder-Cycloalkenyl, Nieder-Alkenyl, Allenyl, Nieder-Alkynyl, $C_2$—$C_{16}$-Alkanoyl, Halo-niederalkanoyl, Aryl-niederalkanoyl, N,N-di(Nieder-Alkyl)sulfamoyl, 1,2,3,4-Tetrahydronaphthyl, Arylthio, Aryl oder Aroyl bedeutet;

oder $R^1$ und $R^2$ zusammen mit dem Stickstoffatom, an welches sie gebunden sind, eine gesättigte 5- oder 6-gliedrige heterocyclische Gruppe mit gegebenenfalls einem weiteren Heteroatom ausgewählt unter O, S und N bilden, wobei das N-Atom ein Wasserstoffatom oder eine niedere Alkyl- oder Arylgruppe trägt und die genannte heterocyclische Gruppe gegebenenfalls an einem Kohlenstoffatom substituiert ist durch 1 oder 2 Nieder-Alkylgruppen;

(b) X die Bedeutung von O, S, SO oder $SO_2$ hat;

(c) Y Halogen, Nieder-Alkyl, Tri(Nieder-Alkyl)silyl, Nieder-Alkoxy, oder eine Gruppe der Formel $R^3S$— oder $R^3SO_2$— bedeutet, worin $R^3$ Nieder-Alkyl oder Aryl ist; n die Bedeutung von 0 oder 1 hat; *und*

(d) R Nieder-Alkyl, Nieder-Alkenyl, Nieder-Cycloalkyl, Allenyl, Nieder-Alkoxy-niederalkyl, Arylniederalkyl, Aryl, eine heteroaromatische oder heteroaromatische-Phenylgruppe bedeutet, mit der Maßgabe, daß wenn X $SO_2$ ist R auch —$NR^1R^2$ sein kann, wobei $R^1$ und $R^2$ die obige Bedeutung haben; der Begriff "Nieder" in Verbindung mit einer Alkyl-, Alkoxy-, Alkanoyl-, Alkenyl- oder Alkynylgruppe bedeutet, daß die Gruppe bis zu 6 Kohlenstoffatomen aufweist; "Nieder" in Verbindung mit einer Cycloalkyl-Gruppe bedeutet, daß die Gruppe 3 bis 7 Kohlenstoffatome aufweist; "Nieder" in Verbindung mit einer Cycloalkenylgruppe bedeutet, daß die Gruppe 4 bis 7 Kohlenstoffatome aufweist; und der Begriff "Aryl" unsubstituiertes Phenyl, unsubstituiertes Naphthyl oder substituiertes Phenyl bedeutet.

2. Verbindung gemäß Anspruch 1, worin substituiertes Phenyl entweder (a) Phenyl substituiert mit 1 oder 2 Substituenten ausgewählt unter Nieder-Alkyl, Nieder-Alkoxy, Hydroxy, Halogen, Trifluoromethyl, Cyano, Amino, Di(Nieder-Alkyl)amino, Nitrol, Nieder-Alkylthio, Nieder-Alkylsulphinyl, Nieder-Alkylsulphonyl, Thiocarbamoyl, Isothiocyanato, Methylendioxy, Sulphamoyl, N,N-Di(Nieder-Alkyl)-sulphamoyl, und N-(Nieder-Alkyl)carbamoyloxy, (b) Pentafluorophenyl, (c) Biphenylyl oder (d) Phenyl monosubstituiert durch Phenoxy, Cyanophenoxy, Halophenoxy, Nitrophenoxy, Dinitrophenoxy, Pentafluorophenoxy, oder Halophenylthio bedeutet.

3. Verbindung gemäß Anspruch 1 oder 2, wobei unter heteroaromatisch verstanden wird Thienyl, Pyridyl, Imidazolyl, Benzthiazolyl, Benzoxazolyl, Pyrimidinyl, Chinolyl, Thiazolyl oder Pyridazinyl, gegebenenfalls enthaltend einen oder zwei Substituenten ausgewählt unter Nieder-Alkyl, Nieder-Alkoxy und Halogen.

4. Verbindung gemäß Anspruch 1, 2 oder 3, worin im Falle, daß $R^1$ und $R^2$ zusammen mit dem Stickstoffatom, an welches sie gebunden sind, eine heterocyclische Gruppe wie in Anspruch 1 definiert bilden, diese Gruppe eine 1-Pyrrolidinyl-, Piperidino-, Morpholino- oder Piperazino-Gruppe bedeuten, wobei die Morpholinogruppen gegebenenfalls substituiert ist durch eine oder zwei Methylgruppen und die Piperazino-Gruppe gegebenenfalls substituiert ist in 4-Stellung durch eine Phenyl-Gruppe, die ihrerseits substituiert sein kann durch 1 oder 2 Halogenatome oder eine $CF_3$-Gruppe.

5. Verbindung gemäß Anspruch 1, worin

(a) $R^1$ die Bedeutung von Wasserstoff oder $C_1$—$C_4$-Alkyl besitzt;

und $R^2$ $C_1$—$C_4$-Alkyl, Methoxy, 2,2,2-Trifluoroethyl, Hydroxymethyl, 2-Methoxyethoxymethyl, $C_1$—$C_4$-Alkoxycarbonylmethyl, $C_2$—$C_5$-Alkanoyloxymethyl, Benzyl; Benzyl mono- oder disubstituiert an dem Phenylring durch $C_1$—$C_4$-Alkyl oder Halogen oder monosubstituiert durch Methylendioxy; Pentafluorophenylmethyl, Phenethyl, Phenoxy-($C_1$—$C_4$-Alkyl), Thiocyanatoacetyl, Cyclopropylmethyl, $C_3$—$C_6$-Cycloalkyl, Cyclohexenyl, Allyl, Allenyl, Propargyl, $C_2$—$C_{16}$-Alkanoyl, Haloacetyl, Phenylacetyl, N,N-Dimethylsulphamoyl, 1,2,3,4-Tetrahydronaphth-1-yl, Phenylthio, Benzoyl gegebenenfalls mono- oder disubstituiert durch $C_1$—$C_4$-Alkyl, $C_1$—$C_4$-Alkoxy oder Halogen; oder Phenyl bedeutet;

oder $R^1$ und $R^2$ zusammengenommen mit dem Stickstoffatom an welches sie gebunden sind 1-Pyrrolidinyl, Piperidino, Morpholino oder Piperazino darstellen, wobei die Morpholinogruppe gegebenenfalls substituiert ist durch 1 oder 2 Methylgruppen und die Piperazinogruppe gegebenenfalls substituiert ist in 4-Stellung durch eine Phenylgruppe, die ihrerseits substituiert sein kann durch 1 oder 2 Halogenatome oder die $CF_3$-Gruppe;

(b) X die Bedeutung von O, S, SO oder $SO_2$ besitzt;

(c) Y Halogen, $C_1$—$C_4$-Alkyl- Trimethylsilyl, $C_1$—$C_4$-Alkoxy oder eine Gruppe der Formel $R^3S$ bedeutet, worin $R^3$ $C_1$—$C_4$-Alkyl oder Halophenyl darstellt; n die Bedeutung von 0 oder 1 hat; *und*

(d) R die Bedeutung $C_1$—$C_4$-Alkyl, Allyl, $C_3$—$C_6$-Cycloalkyl, Allenyl, Methoxymethyl, Phenethyl, Naphthyl, Biphenyl, Thienyl, Pyridyl, Halopyridyl, Dihaloimidazolyl, Chinolyl, Benzoxazolyl, Benzthiazolyl, Methoxypyridazinyl, Phenyl; Phenyl mono- oder disubstituiert durch $C_1$—$C_4$-Alkyl, $C_1$—$C_4$-Alkoxy, Halogen, Cyano, Trifluormethyl, Hydroxy, Sulphamoyl, N,N-Dimethylsulphamoyl, Amino, Dimethylamino, Nitro, Methylthio, Methylsulphinyl, Methylsulphonyl, N-Methylcarbamoyloxy, Isothiocyanato oder Thiocarbamoyl; oder Phenyl monosubstituiert durch Phenoxy, Pentafluorophenoxy, Cyanophenoxy, Nitrophenoxy, Dinitrophenoxy, Halophenoxy, Halophenylthio oder 4-Methyl-2-thiazolyl besitzt; mit der Maßgabe, daß wenn X $SO_2$ darstellt, R auch —$NR^1R^2$ sein kann, worin $R^1$ $C_1$—$C_4$-Alkyl und $R^2$ $C_1$—$C_4$-Alkyl, Cyclopropyl oder Allyl darstellt, oder $R^1$ und $R^2$ zusammengenommen mit dem Stickstoffatom an welches sie gebunden sind eine Morpholinogruppe bedeuten, die gegebenenfalls substituiert ist durch 1 oder 2 Methylgruppen.

6. Verbindung gemäß Anspruch 1 der Formel

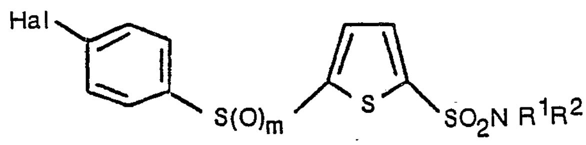

--- (IA)

worin $R^1$ Wasserstoff oder $C_1$—$C_4$-Alkyl, $R^2$ $C_1$—$C_4$-Alkyl, $C_3$—$C_6$-Cycloalkyl, $C_2$—$C_4$-Alkanoyl oder Benzoyl, "Hal" Cl oder Br und m 0 oder 2 darstellt.

7. Verbindung gemäß Anspruch 1 der Formel

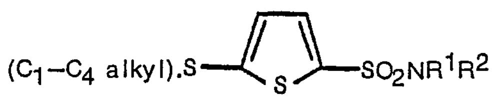

--- (IB)

worin $R^1$ und $R^2$ die in Anspruch 6 gegebene Bedeutung haben.

8. Verbindung gemäß Anspruch 1 der Formel

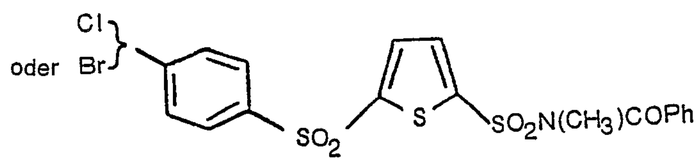

9. Verbindung der Formel (I) gemäß Anspruch 1 und deren Alkali- oder nicht-toxischen Säureadditionssalze,
worin

(a) $R^1$ Wasserstoff oder Nieder-Alkyl bedeutet;

und $R^2$ die Bedeutung Nieder-Alkyl, Nieder-Alkoxycarbonyl-niederalkyl, Nieder-Alkanoyloxy-niederalkyl, Aryl-niederalkyl, Nieder-Cycloalkyl-niederalkyl, Nieder-Cycloalkyl, Nieder-Cycloalkenyl, Nieder-Alkenyl, Allenyl, Nieder Alkynyl, Nieder-Alkanoyl, Halo-niederalkanoyl, Aryl, Aroyl, Arylthio oder Nieder-Alkoxy besitzt; oder $R^1$ und $R^2$ zusammen mit dem Stickstoffatom an welches sie gebunden sind eine gesättigte 5- oder 6-gliedrige heterocyclische Gruppe darstellen, die gegebenenfalls ein weiteres Heteroatom enthält ausgewählt aus O, S und N, wobei das N-Atom ein Wasserstoffatom oder eine Nieder-Alkyl- oder Arylgruppe trägt und die heterocyclische Gruppe gegebenenfalls substituiert ist an einem Kohlenstoffatom durch 1 oder 2 Nieder-Alkylgruppen;

(b) X die Bedeutung von O, S, SO oder $SO_2$ hat;

(c) Y Halogen, Nieder-Alkyl, Nieder-Alkoxy, Tri(Nieder-Alkyl)silyl oder eine Gruppe der Formel $R^3S$— oder $R^3SO_2$— bedeutet, worin $R^3$ Nieder-Alkyl oder Aryl ist; n 0 oder 1 bedeutet *und*

(d) R Nieder-Alkyl, Nieder-Alkenyl, Allenyl, Nieder-Alkoxy-niederalkyl, Aryl-niederalkyl, Aryl oder eine heteroaromatische Gruppe ist, mit der Maßgabe, daß wenn X $SO_2$ ist, R auch —$NR^1R^2$ sein kann, worin $R^1$ und $R^2$ die obige Bedeutung haben, "Aryl" für unsubstituiertes Phenyl, unsubstituiertes Naphthyl oder Phenyl substituiert durch 1 oder 2 Substituenten ausgewählt unter Nieder-Alkyl, Nieder-Alkoxy, Hydroxy, Halogen, Trifluormethyl, Cyano, Nitro, Nieder-Alkyl-thio, Nieder-Alkylsulphinyl, Nieder-Alkylsulphonyl und N,N-Di(Nieder-Alkyl)sulphamoyl steht;

und "nieder" die in Anspruch 1 angegebene Bedeutung besitzt.

10. Verfahren zur Herstellung einer Verbindung der Formel (I) gemäß Anspruch 1 worin X für S steht, wobei eine Verbindung der Formel

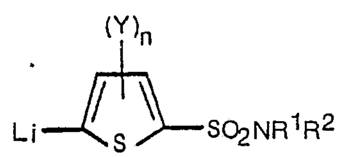

worin Y, $R^1$, $R^2$ und n die in Anspruch 1 angegebene Bedeutung besitzen, mit einem Disulfid der Formel

RS—SR

worin R die in Anspruch 1 angegebene Bedeutung besitzt, umgesetzt wird.

11. Verfahren zur Herstellung einer Verbindung der Formel (I) gemäß Anspruch 1, worin X für O oder S steht und R eine Aryl- oder heteroaromatische Gruppe darstellt, wobei in einem organischen Lösungsmittel und in Anwesenheit einer Base eine Verbindung der Formel

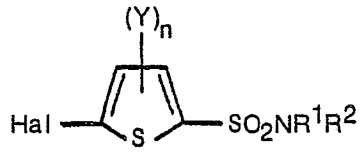

worin Y, $R^1$, $R^2$ und n die in Anspruch 1 angegebene Bedeutung besitzen und "Hal" Br, Cl oder J darstellen, mit einer Verbindung der Formel

RXH

worin R und X die hier gegebene Definition besitzen, umgesetzt wird.

12. Verfahren zur Herstellung einer Verbindung der Formel (I) gemäß Anspruch 1, worin $R^2$ etwas anderes bedeutet als Aryl und $R^1$ und $R^2$ zusammen mit dem Stickstoffatom an welches sie gebunden sind, nicht einen Ring bilden, wobei in Anwesenheit einer Base eine Verbindung der Formel

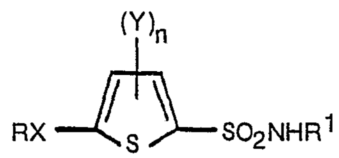

mit einer Verbindung der Formel $R^2$.Hal umgesetzt wird, wobei R, X, Y und n die in Anspruch 1 angegebene Bedeutung besitzen, $R^1$ und $R^2$ die in dieser Methode angegebene Bedeutung besitzen und "Hal" für Br, Cl oder J steht.

13. Verfahren zur Herstellung einer Verbindung der Formel (I) gemäß Anspruch 1 worin $R^1$ Nieder-Alkyl darstellt, wobei die entsprechende Verbindung der Formel (I), worin $R^1$ H bedeutet, mit einem Nieder-Alkylhalogenid in Anwesenheit einer Base umgesetzt wird.

14. Verfahren zur Herstellung einer Verbindung der Formel (I) gemäß Anspruch 1, worin $R^1$ und $R^2$ beide Nieder-Alkyl darstellen, wobei die entsprechende Verbindung der Formel (I) worin $R^1$ und $R^2$ beide Wasserstoff darstellen, mit mindestens 2 Äquivalenten eines Nieder-Alkylhalogenids in Anwesenheit einer Base umgesetzt wird.

15. Verfahren zur Herstellung einer Verbindung der Formel (I) gemäß Anspruch 1, worin X für S steht, wobei eine Verbindung der Formel

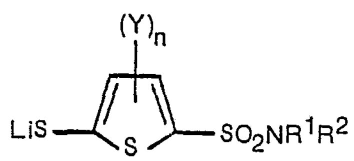

mit einer Verbindung der Formel R.Hal umgesetzt wird, wobei Hal Br, Cl oder J bedeutet und R, $R^1$, $R^2$, Y und n die in Anspruch 1 angegebene Bedeutung besitzen.

16. Verfahren zur Herstellung einer Verbindung der Formel (I) gemäß Anspruch 1, worin X SO oder $SO_2$ bedeutet, wobei die entsprechende Verbindung, in welcher X S darstellt, oxidiert wird unter Verwendung von m-Chloroperbenzoesäure bzw. Wasserstoffperoxid in Eisessig.

17. Verfahren zur Herstellung einer Verbindung der Formel (I) gemäß Anspruch 1 wobei eine Verbindung der Formel

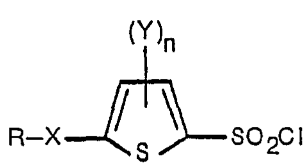

mit einem Amin der Formel —$R^1R^2NH$ umgesetzt wird, wobei R, $R^1$, $R^2$, X, Y und n die in Anspruch 1 angegebene Bedeutung besitzen.

18. Ein insektizides oder larvizides Mittel oder Konzentrat enthaltend eine Verbindung der Formel (I) oder ein Alkali- oder nicht-toxisches Säureadditionssalze davon gemäß Anspruch 1 zusammen mit einem nicht-toxischen Verdünnungsmittel oder Träger.

## Revendications

1. Composés de formule:

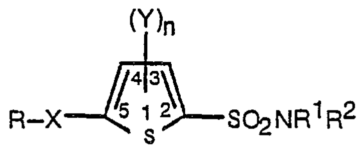

--- (I)

et leurs sels de métaux alcalins ou leurs sels d'addition d'acides non toxiques, formule dans laquelle:

(a) $R^1$ est l'hydrogène ou un groupe alkyle inférieur;

et $R^2$ est un groupe alkyle inférieur, alkoxy inférieur, 2,2,2-trifluoréthyle, hydroxy-alkyle inférieur, 2-méthoxyéthoxyméthyle, (alkoxy inférieur)-carbonylalkyle inférieur, (alcanoyloxy inférieur)-alkyle inférieur, aryl-alkyle inférieur, aryloxy-alkyle inférieur, thiocyanatoacétyle, (cycloalkyle inférieur)-alkyle inférieur, cycloalkyle inférieur, cycloalcényle inférieur, alcényle inférieur, allényle, alcynyle inférieur, alcanoyle en $C_2$ à $C_{16}$, halogénalcanoyle inférieur, aryl-alcanoyle inférieur, N,N-di-(alkyle inférieur)-sulfamoyle, 1,2,3,4-tétrahydronaphtyle, arylthio, aryle ou aroyle;

ou bien $R^1$ et $R^2$ forment conjointement avec l'atome d'azote auquel ils sont liés un groupe hétérocyclique pentagonal ou hexagonal saturé renfermant éventuellement un autre hétéro-atome choisi entre O, S et N, ledit atome d'azote portant un atome d'hydrogène ou un groupe alkyle inférieur ou aryle, ledit groupe hétérocyclique étant éventuellement substitué sur un atome de carbone par un ou deux groupes alkyle inférieurs;

(b) X représente O, S, SO ou $SO_2$;

(c) Y est un halogène, un groupe alkyle inférieur, tri-(alkyle inférieur)-silyle, alkoxy inférieur ou un groupe de formule $R^3S$— ou $R^3SO_2$— dans laquelle $R^3$ est un radical alkyle inférieur ou aryle; *n* est égal à 0 ou à 1; *et*

(d) R est un groupe alkyle inférieure, alcényle inférieur, cycloalkyle inférieur, allényle, (alkoxy inférieur)-alkyle inférieur, aryl-alkyle inférieur, aryle, hétéro-aromatique ou hétéro-aromatique-phényle, sous réserve que lorsque X représente $SO_2$, R puisse également représenter —$NR^1R^2$ où $R^1$ et $R^2$ ont les définitions données ci-dessus;

le terme "inférieur" appliqué à un groupe alkyle, alkoxy, alcanoyle, alcényle ou alcynyle signifiant que ce groupe comprend jusqu'à 6 atomes de carbone; le terme "inférieur" appliqué à un groupe cycloalkyle signifiant que ce groupe comprend 3 à 7 atomes de carbone; le terme "inférieur" appliqué à un groupe cycloalcényle signifiant que ledit groupe comprend 4 à 7 atomes de carbone;

et le terme "aryle" signifie un groupe phényle non substitué, naphtyle non substitué ou phényle substitué.

2. Composé suivant la revendication 1, dans lequel le groupe phényle substitué est (a) un groupe phényle substitué par un ou deux substituants choisis entre des radicaux alkyle inférieur, alkoxy inférieur, hydroxy, halogéno, trifluorométhyle, cyano, amino, di-(alkyle inférieur)-amino, nitro, alkylthio inférieur, alkylsulfinyle inférieur, alkylsulfonyle inférieur, thiocarbamoyle, isothiocyanato, méthylènedioxy, sulfamoyle, N,N-di-(alkyle inférieur)-sulfamoyle et N-(alkyle inférieur)-carbamoyloxy, (b) un groupe pentafluorophényle, (c) un groupe biphénylyle ou (d) un groupe phényle monosubstitué par un reste phénoxy, cyanophénoxy, halogénophénoxy, nitrophénoxy, dinitrophénoxy, pentafluorophénoxy ou halogénophénylthio.

3. Composé suivant la revendication 1 ou 2, dans lequel on entend désigner par hétéroaromatique un groupe thiényle, pyridyle, imidazolyle, benzothiazolyle, benzoxazolyle, pyrimidinyle, quinolyle, thiazolyle ou pyridazinyle, portant éventuellement un ou deux substituants choisis entre des radicaux alkyle inférieur, alkoxy inférieur et halogéno.

4. Composé suivant la revendication 1, 2 ou 3, dans lequel $R^1$ et $R^2$ forment conjointement avec l'atome d'azote auquel ils sont liés un groupe hétérocyclique tel que défini dans la revendication 1, ce groupe est un groupe 1-pyrrolidinyle, pipéridino, morpholino ou pipérazino, ledit groupe morpholino étant éventuellement substitué par un ou deux groupes méthyle, et ledit groupe pipérazino étant éventuellement substitué en position 4 par un groupe phényle qui peut lui-même être substitué par 1 ou 2 halogènes ou groupes $CF_3$.

5. Composé suivant la revendication 1, dans lequel

(a) $R^1$ est l'hydrogène ou un groupe alkyle $C_1$ à $C_4$;

et $R^2$ est un groupe alkyle en $C_1$ à $C_4$, méthoxy, 2,2,2-trifluoréthyle, hydroxyméthyle, 2-méthoxyéthoxyméthyle, (alkoxy en $C_1$ à $C_4$)-carbonylméthyle, (alcanoyloxy en $C_2$ à $C_5$)-méthyle, benzyle;

benzyle mono- ou di-substitué sur le noyau phényle par un radical alkyle en $C_1$ à $C_4$ ou halogéno ou mono-substitué par un radical méthylènedioxy; pentafluorophénylméthyle, phénéthyle, phénoxy-(alkyle en $C_1$ à $C_4$), thiocyanatoacétyle, cyclopropylméthyle, cycloalkyle en $C_3$ à $C_6$, cyclohexényle, allyle, allényle, propargyle, alcanoyle en $C_2$ à $C_{16}$, halogénacétyle, phénylacétyle, N,N-diméthylsulfamoyle, 1,2,3,4-tétrahydronapht-1-yle, phénylethio, benzoyle éventuellement mono- ou di-substitué par des radicaux alkyle en $C_1$ à $C_4$, alkoxy en $C_1$ à $C_4$ ou halogéno; ou phényle;

ou bien $R^1$ et $R^2$ forment conjointement avec l'atome d'azote auquel ils sont liés un groupe 1-pyrrolidinyle, pipéridino, morpholino ou pipérazino, ledit groupe morpholino étant éventuellement substitué par un ou deux groupes méthyle et ledit groupe pipérazino étant éventuellement substitué en position 4 par un groupe phényle qui peut lui-même être substitué par 1 ou 2 atomes d'halogènes ou groupes $CF_3$;

(b) X représente O, S, SO ou $SO_2$;

(c) Y est un halogène ou un groupe alkyle en $C_1$ à $C_4$, triméthylsilyle, alkoxy en $C_1$ à $C_4$ ou un groupe de formule $R^3S$—, dans laquelle $R^3$ est un radical alkyle en $C_1$ à $C_4$ ou halogénophényle; *n* est égal à 0 ou à 1; *et*

(d) R est un groupe alkyle en $C_1$ à $C_4$, allyle, cycloalkyle en $C_3$ à $C_6$, allényle, méthoxyméthyle, phénéthyle, naphtyle, biphényle, thiényle, pyridyle, halogénopyridyle, dihalogénimidazolyle, quinolyle, benzoxazolyle, benzothiazolyle, méthoxypyridazinyle, phényle; phényle mono- ou di-substitué par un radical alkyle en $C_1$ à $C_4$, alkoxy en $C_1$ à $C_4$, halogéno, cyano, trifluorométhyle, hydroxy, sulfamoyle, N,N-diméthylsulfamoyle, amino, diméthylamino, nitro, méthylthio, méthylsulfinyle, méthylsulfonyle, N-méthylcarbamoyloxy, isothiocyanato ou thiocarbamoyle; ou phényle monosubstitué par un radical phénoxy, pentafluorophénoxy, cyanophénoxy, nitrophénoxy, dinitrophénoxy, halogénophénoxy, halogénophénylthio ou 4-méthyl-2-thiazolyle; sous réserve que lorsque X représente $SO_2$, R puisse également être un groupe —$NR^1R^2$ dans lequel $R^1$ est un radical alkyle en $C_1$ à $C_4$ et $R^2$ est un radical alkyle en $C_1$ à $C_4$, cyclopropyle ou allyle, ou bien $R^1$ et $R^2$ forment conjointement avec l'atome d'azote auquel ils sont liés un groupe morpholino éventuellement substitué par 1 ou 2 groupes méthyle.

6. Composé suivant la revendication 1, de formule:

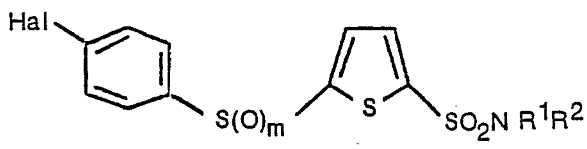

--- (IA)

dans laquelle $R^1$ représente l'hydrogène ou un groupe alkyle en $C_1$ à $C_4$ $R^2$ est un groupe alkyle en $C_1$ à $C_4$, cycloalkyle en $C_3$ à $C_6$, alcanoyle en $C_2$ à $C_4$ ou benzoyle, "Hal" représente Cl ou Br et *m* a la valeur 0 ou 2.

7. Composé suivant la revendication 1, de formule:

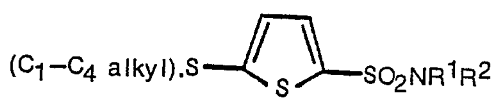

--- (IB)

dans laquelle $R^1$ et $R^2$ ont les définitions données dans la revendication 6.

8. Composé suivant la revendication 1, de formule:

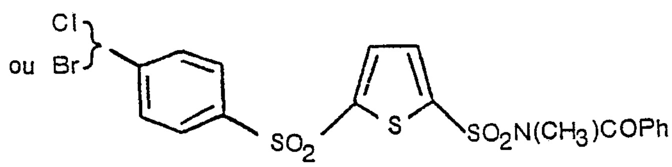

9. Composé de formule (I) suivant la revendication 1 et ses sels de métaux alcalins ou ses sels d'addition d'acides non toxiques:

formule dans laquelle:

(a) $R^1$ est l'hydrogène ou un groupe alkyle inférieur;

et $R^2$ est un groupe alkyle inférieur, (alkoxy inférieur)-carbonyl-alkyle inférieur, (alcanoyloxy inférieur)-alkyle inférieur, aryl-alkyle inférieur, (cycloalkyle inférieur)-alkyle inférieur, cycloalkyle inférieur, cycloalcényle inférieur, alcényle inférieur, allényle, alcynyle inférieur, alcanoyle inférieur, halogénalcanoyle inférieur, aryle, aroyle, arylthio ou alkoxy inférieur;

ou bien $R^1$ et $R^2$ forment conjointement avec l'atome d'azote aquel ils sont liés un groupe hétérocyclique pentagonal ou hexagonal saturé renfermant éventuellement un autre hétéro-atome choisi entre O, S et N, ledit atome d'azote portant un atome d'hydrogène ou un groupe alkyle inférieur ou aryle, ledit groupe hétérocyclique étant éventuellement substitué sur un atome de carbone par 1 ou 2 groupes alkyle inférieurs;

(b) X représente O, S, SO ou $SO_2$;

(c) Y est un halogène, un groupe alkyle inférieur, alkoxy inférieur, tri-(alkyle inférieur)-silyle ou un groupe de formule $R^3S$— ou $R^3SO_2$— dans laquelle $R^3$ est un radical alkyle inférieur ou aryle; *n* est égal à 0 ou à 1; *et*

(d) R est un groupe alkyle inférieur, alcényle inférieurr, allényle, (alkoxy inférieur)-alkyle inférieur, aryl-alkyle inférieur, aryle ou hétéro-aromatique, sous réserve que lorsque X représente $SO_2$, R puisse également être un groupe —$NR^1R^2$ dans lequel $R^1$ et $R^2$ ont les définitions données ci-dessus; "aryle" désigne un groupe phényle non substitué, naphtyle non substitué ou phényle substitué par 1 ou 2 substituants choisis entre des substituants alkyle inférieur, alkoxy inférieur, hydroxy, halogéno, trifluorométhyle, cyano, nitro, alkylthio inférieur, alkylsulfinyle inférieur, alkylsulfonyle inférieur et N,N-di-(alkyle inférieur)-sulfamoyle;

et "inférieur" a la définition donnée dans la revendication 1.

10. Procédé de préparation d'un composé de formule (I) suivant la revendication 1, dans laquelle X représente S, qui consiste à faire réagir un composé de formule:

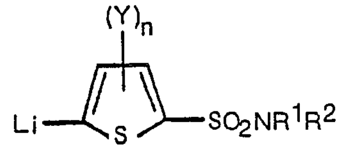

dans laquelle Y, $R^1$, $R^2$ et *n* ont les définitions données dans la revendication 1,

avec un disulfure de formule:

RS—SR

dans laquelle R a la définition donnée dans la revendication 1.

11. Procédé de préparation d'un composé de formule (I) suivant la revendication 1, dans laquelle X représente O, S et R est un groupe aryle ou un groupe hétéro-aromatique, qui consiste à faire réagir, dans un solvant organique et en présence d'une base, un composé de formule:

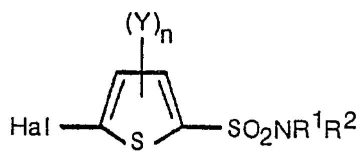

dans laquelle Y, $R^1$, $R^2$ et *n* ont la définition donnée dans la revendication 1 et "Hal" représente Br, Cl ou I, avec un composé de formule:

RXH

dans laquelle R et X ont les définitions données dans ce procédé.

12. Procédé de préparation d'un composé de formule (I) suivant la revendication 1, dans laquelle $R^2$ représente autre chose qu'un groupe aryle et $R^1$ et $R^2$, pris conjointement avec l'atome d'azote auquel ils sont liés, ne forment pas un noyau, procédé qui consiste à faire réagir, en présence d'un base, un composé de formule:

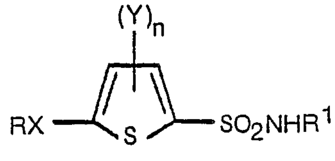

avec un composé de formule $R^2$. Hal

dans laquelle R, X, Y et *n* ont les définititions données dans la revendication 1, $R^1$ et $R^2$ ont les définitions données dans ce procédé et "Hal" représente Br, Cl ou I.

13. Procédé de préparation d'un composé de formule (I) suivant la revendication 1, dans laquelle $R^1$ est un groupe alkyle inférieur, qui consiste à faire réagir le composé correspondant de formule (I) dans laquelle $R^1$ représente H avec un halogénure d'alkyle inférieur en présence d'une base.

14. Procédé de préparation d'un composé de formule (I) suivant la revendication 1, dans laquelle $R^1$ et $R^2$ représentent chacun un groupe alkyle inférieur, qui consiste à faire réagir le composé correspondant de formule (I) dans laquelle $R^1$ et $R^2$ représentent chacun un atome d'hydrogène avec au moins 2 équivalents d'un halogénure d'alkyle inférieur en présence d'une base.

15. Procédé de préparation d'un compose de formule (I) suivant la revendication 1, dans laquelle X représente S, qui consiste à faire réagir un composé de formule:

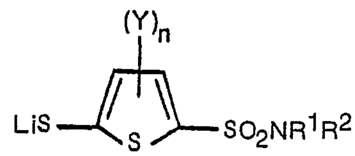

avec un composé de formule R.Hal

où Hal représente Br, Cl ou I et R, $R^1$, $R^2$, Y et *n* ont les définitions données dans la revendication 1.

16. Procédé de préparation d'un composé de formule (I) suivant la revendication 1, dans laquelle X représent SO ou $SO_2$, qui consiste à oxyder le composé correspondant dans lequel X représente S en utilisant, respectivement, l'acide *m*-chloroperbenzoïque ou le peroxyde d'hydrogène dans l'acide acétique cristallisable.

17. Procédé de préparation d'un composé de formule (I) suivant la revendication 1, qui consiste à faire réagir un composé de formule:

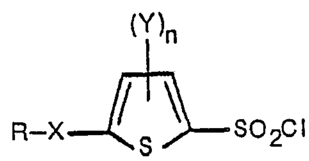

avec une amine de formule: $R^1R^2NH$,

R, $R^1$, $R^2$, X, Y et *n* ayant les définitions données dans la revendication 1.

18. Composition ou concentré insecticide ou larvicide comprenant un composé de formule (I) ou un sel de métal alcalin ou un sel d'addition d'acide non toxique de ce composé suivant la revendication 1, en association avec un diluant ou support non toxique.